# EUROPEAN PATENT APPLICATION

(11) **EP 2 343 365 A1**
(43) Date of publication of application: **13.07.2011**
(21) Application number: 10186114.4
(22) Date of filing: 04.10.2002
(51) Int. Cl.: C12N 9/12, C12N 15/00, C12N 5/00, G01N 33/50

(54) **Mutated Abl kinase domains**

(30) Priority: 05.10.2001 US 327389 P; 12.10.2001 US 328740 P; 11.01.2002 US 347351 P
(62) Divisional of application: 08166771.9
(71) Applicant: Novartis AG, 4056 Basel (CH); Université Victor Segalen Bordeaux 2, 33076 Bordeaux Cedex (FR); Oregon Health and Science University, Portland, OR 97201-4753 (US); Technische Universität München, 80333 München (DE); University of Heidelberg, 69117 Heidelberg (DE); Centre Hospitalier Regional Universitaire de Lille, 59037 Lille Cédex (FR); MEDVET SCIENCE PTY. LTD., Stepney, South Australia 5069 (AU)
(72) Inventor: Barthe, Christophe, 33127, Martignas Sur Jalles (FR); Branford, Susan, Flagstaff Hill, South Australia 5159 (AU); Corbin, Amie, Portland, OR 97219 (US); Druker, Brian Jay, Portland, OR 97239 (US); Duyster, Justus, 81739, Munich (DE); Hochhaus, Andreas, 68163, Mannheim (DE); Hughes, Timothy, Kensington Gardens, South Australia 5068 (AU); Kreil, Sebastian, 68165, Mannheim (DE); Leguay, Thibaut, 33000, Bordeaux (FR); Mahon, Francois-Xavier, 33000, Bordeaux (FR); Marit, Gerald, 33200, Bordeaux (FR); Mueller, Martin, 69120, Heidelberg (DE); Peschel, Christian, 81739, Munich (DE); Preudhomme, Claude, 62150, Houdain (FR); Roche Lestienne, Catherine, 59800, Lille (FR); Rudzki, Zbigniew, Rostrevor, South Australia 5073 (AU)
(74) Representative: Kassow, Anders

(57) **Abstract**

The present invention relates to isolated polypeptides which comprise a functional kinase domain comprising the amino acid sequence of the native human Abl kinase domain or an essentially similar sequence thereof in which at least one amino acid selected from Met244, Leu248, Gly250, Glu252, Tyr253, Val256, Glu258, Phe311, Ile313, Phe317, Met318, Met351, Glu355, Glu359, Ile360, His361, Leu370, Asp381, Phe382, His396, Ser417, Glu459 and Phe486 is replaced by another amino acid, said mutated functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-y(methyl)-benzamide or a salt thereof, to the use of such polypeptides to screen for compounds which inhibit the tyrosine kinase activity of such polypeptides, to nucleic acid molecules encoding such polypeptides, to recombinant vectors and host cells comprising such nucleic acid molecules and to the use of such nucleic acid molecules in the production of such polypeptides for use in screening for compounds which inhibit the tyrosine kinase activity of such polypeptides.

## Description

### Field of the Invention:

This invention relates to isolated polypeptides which comprise a functional kinase domain comprising the amino acid sequence of the native human Abl kinase domain or an essentially similar sequence thereof in which at least one amino acid selected from Met244, Leu248, Gly250, Glu252, Tyr253, Val256, Glu258, Phe311, Ile313, Phe317, Met318, Met351, Glu355, Glu359, Ile360, His361, Leu370, Asp381, Phe382, His396, Ser417, Glu459 and Phe486 is replaced by another amino acid, said mutated functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof, to the use of such polypeptides to screen for compounds which inhibit the tyrosine kinase activity of such polypeptides, to nucleic acid molecules encoding such polypeptides, to recombinant vectors and host cells comprising such nucleic acid molecules and to the use of such nucleic acid molecules in the production of such polypeptides for use in screening for compounds which inhibit the tyrosine kinase activity of such polypeptides.

### Background of the Invention:

Bcr-Abl, a constitutively activated tyrosine kinase resulting from the formation of the Philadelphia chromosome [Nowell P.C. and Hungerford D.A., Science 132, 1497 (1960)] by reciprocal translocation between the long arms of chromosomes 9 and 22 [Rowley J.D., Nature 243, 290-293 (1973)], has been established as the characteristic molecular abnormality present in virtually all cases of chronic myeloid leukemia (CML) and up to 20 percent of adult acute lymphoblastic leukemia (ALL) [Faderl S. et al., N Engl J Med 341, 164-172 (1999); Sawyers C.L., N Engl J Med 340, 1330-1340 (1999)]. Bcr-Abl is sufficient to cause CML in mice [Daley G.Q. et al., Science 247, 824-830 (1990)] and its transforming capacity is absolutely dependent on tyrosine kinase activity [Lugo T.G. et al., Science 247, 1079 (1990)]. The compound N-[4-methyl-3-(4-pyridin-3-yl-pyrimidin-2-ylamino)-phenyl]-4-(4-methyl-piperazin-1-ylmethyl)-benzamide (hereinafter also referred to as "STI571"; STI571 is described in EP 0 564 409 and, in the form of the methane sulfonate salt, in WO 99/03854), a competitive inhibitor at the ATP-binding site of Bcr-Abl, as well as of the receptor for platelet-derived growth factor, and c-kit tyrosine kinase [Lugo T.G. et al., Science 247, 1079 (1990)], has been shown to be capable of very rapidly reversing the clinical and hematological abnormalities of CML in chronic phase and in blast crisis as well as of Ph chromosome-positive (Ph+) acute lymphoblastic leukemia (Ph+ ALL) [Druker B.J. et al., N Engl J Med 344, 1031-1037 (2001); Druker B.J. et al., N Engl J Med 344, 1038-1042 (2001)]. Whereas almost all chronic phase CML patients durably respond, remissions in CML blast crisis and Ph+ ALL are transient, and most patients relapse after several months, despite continued therapy with STI571 [Druker B. J. et al., N Engl J Med 344, 1038-1042 (2001)]. The mechanism of resistance to STI571 is subject of intense research. It was now surprisingly found that mutations present in the kinase domain of the Bcr-Abl gene of patients suffering from CML or Ph+ ALL account for the biological resistance of these patients towards STI571 treatment in that said mutations lead to resistance of the Bcr-Abl tyrosine kinase towards inhibition by STI571.

These findings are extremely valuable in e.g. finding new compounds or combinations of compounds which are capable to overcome resistance towards treatment with STI571. Moreover, knowledge of such mutations is also very useful in the diagnosis of Ph+ leukemias in that it allows e.g. the detection of drug-resistant clones before clinical relapse of the patient.

### Definitions:

Within the context of this disclosure the following expressions, terms and abbreviations have the meanings as defined below:
In the expression "a functional kinase domain", the term "functional" indicates that the respective kinase domain possesses tyrosine kinase activity. Preferably, the kinase activity of such a functional kinase domain is in the range of that of the native human Abl kinase domain.
In the expression "a functional kinase domain being resistant to inhibition of its tyrosine kinase activity by STI571 or a salt thereof', the term "resistant" means that STI571 inhibits the respective functional kinase domain with an IC₅₀ that is higher than that of the native human Abl kinase domain, i.e. higher than about 0.025 µM, preferably higher than about 0.15 µM, more preferably higher than about 0.25 µM, most preferably higher than about 5 µM.
In the expression "amino acid sequence of the native human Abl kinase domain or an essentially similar sequence thereof", the part "or an essentially similar sequence thereof" refers to the amino acid sequence of the native human Abl kinase domain containing mutations, including amino acid exchanges, amino acid deletions and/or amino acid additions, that are not essential for the functionality of the kinase and its resistance to inhibition by STI571 or a salt thereof within the meaning of the term "functional" and "resistant" as defined hereinabove.

The expression "replaced by another amino acid" refers to the replacement of a certain natural amino acid by another natural amino acid.

The names of the amino acids are either written out or the one letter or three letter codes are used. Mutations are referred to by accepted nomenclature, e.g. "Ala380Thr" or "380 Ala→Thr" both indicating that alanine at position 380 is replaced by threonine.

SEQ ID NO:1 represents the cDNA coding for the native human Abl protein (human c-abl mRNA; GenBank Accession No.: X16416).

SEQ ID NO:2 represents the amino acid sequence of the native human Abl protein (human c-Abl; SwissProt Acc. No.: P00519).

Unless indicated otherwise, the number given for a certain amino acid refers to the numbering of the amino acids in SEQ ID NO:2. In an amino acid sequence that is essentially similar to the amino acid sequence of the native human Abl kinase domain within the meaning as defined above, the amino acids are numbered in accordance with the numbering of the amino acids in SEQ ID NO:2.

The term "isolated" means that the material is removed from its original environment (e.g., the natural environment if it is naturally occurring).

A "host cell", refers to a prokaryotic or eukaryotic cell that contains heterologous DNA that has been introduced into the cell by any means, e.g., electroporation, calcium phosphate precipitation, microinjection, transformation, viral infection, and the like.

### Description of the Invention:

In practicing the present invention, many conventional techniques in molecular biology, microbiology, and recombinant DNA are used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

In particular, the polypeptides of the present invention can be produced by recombinant DNA technology using techniques well-known in the art. Methods which are well known to those skilled in the art can be used to construct expression vectors containing the sequences encoding the polypeptides of the invention and appropriate transcriptional/translational control signals. A variety of host-expression vector systems can be utilized to express the polypeptides of the invention.
(1) The invention relates to an isolated polypeptide which comprises a functional kinase domain comprising the amino acid sequence of the native human Abl kinase domain or an essentially similar sequence thereof in which at least one amino acid selected from Gly250, Tyr253, Val256, Glu258, Ile313, Met318, Leu370, Phe382 and His396 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(2) The invention especially relates to an isolated polypeptide which comprises a functional kinase domain comprising the amino acid sequence of the native human Abl kinase domain or an essentially similar sequence thereof in which at least one amino acid selected from Gly250, Tyr253, Glu258 and His396 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(3) The invention relates to an isolated polypeptide which comprises a functional kinase domain comprising the amino acid sequence of the native human Abl kinase domain or an essentially similar sequence thereof in which at least one amino acid selected from Met244, Gly250, Tyr253, Val256, Glu258, Ile313, Phe317, Met318, Met351, Ile360, His361, Leu370, Asp381, Phe382, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(4) The invention further relates to an isolated polypeptide which comprises a functional kinase domain comprising the amino acid sequence of the native human Abl kinase domain or an essentially similar sequence thereof in which at least one amino acid selected from Met244, Gly250, Tyr253, Val256, Glu258, Ile313, Phe317, Met318, Ile360, His361, Leu370, Asp381, Phe382, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(5) The invention especially relates to an isolated polypeptide which comprises a functional kinase domain comprising the amino acid sequence of the native human Abl kinase domain or an essentially similar sequence thereof in which at least one amino acid selected from Met244, Gly250, Tyr253, Glu258, Phe317, Met351, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(6) The invention relates also especially to an isolated polypeptide which comprises a functional kinase domain comprising the amino acid sequence of the native human Abl kinase domain or an essentially similar sequence thereof in which at least one amino acid selected from Met244, Gly250, Tyr253, Glu258, Phe317, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(7) The invention relates to an isolated polypeptide which comprises a functional kinase domain comprising the amino acid sequence of the native human Abl kinase domain or an essentially similar sequence thereof in which at least one amino acid selected from Met244, Phe317, Met351, Ile360, His361, Asp381, and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof, and wherein optionally at least one additional amino acid selected from Gly250, Tyr253, Val256, Glu258, Ile313, Met318, Leu370, Phe382 and His396 is replaced by another amino acid.
(8) The invention relates to an isolated polypeptide which comprises a functional kinase domain comprising the amino acid sequence of the native human Abl kinase domain or an essentially similar sequence thereof in which the amino acid Phe311 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(9) In a preferred embodiment the invention relates to an isolated polypeptide according to the preceding paragraph (8), wherein optionally at least one additional amino acid selected from Met244, Gly250, Tyr253, Val256, Glu258, Iel313, Phe317, Met318, Met351, Ile360, His361, Leu370, Asp381, Phe382, His396 and Phe486 is replaced by another amino acid.
(10) In a preferred embodiment the invention relates to an isolated polypeptide according to the preceding paragraph (8), wherein optionally at least one additional amino acid selected from Met244, Gly250, Tyr253, Val256, Glu258, Ile313, Phe317, Met318, Ile360, His361, Leu370, Asp381, Phe382, His396 and Phe486 is replaced by another amino acid.
(11) The invention relates to an isolated polypeptide which comprises a functional kinase domain comprising the amino acid sequence of the native human Abl kinase domain or an essentially similar sequence thereof in which at least one amino acid selected from Met244, Gly250, Tyr253, Val256, Glu258, Phe311, Ile313, Phe317, Met318, Met351, Ile360, His361, Leu370, Asp381, Phe382, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(12) The invention further relates to an isolated polypeptide which comprises a functional kinase domain comprising the amino acid sequence of the native human Abl kinase domain or an essentially similar sequence thereof in which at least one amino acid selected from Met244, Gly250, Tyr253, Val256, Glu258, Phe311, Ile313, Phe317, Met318, Ile360, His361, Leu370, Asp381, Phe382, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(13) The invention especially relates to an isolated polypeptide which comprises a functional kinase domain comprising the amino acid sequence of the native human Abl kinase domain or an essentially similar sequence thereof in which at least one amino acid selected from Met244, Gly250, Tyr253, Glu258, Phe311, Phe317, Met351, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(14) The invention relates also especially to an isolated polypeptide which comprises a functional kinase domain comprising the amino acid sequence of the native human Abl kinase domain or an essentially similar sequence thereof in which at least one amino acid selected from Met244, Gly250, Tyr253, Glu258, Phe311, Phe317, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(15) The invention relates very especially to an isolated polypeptide which comprises a functional kinase domain comprising the amino acid sequence of the native human Abl kinase domain or an essentially similar sequence thereof in which at least one amino acid selected from Gly250, Tyr253, Glu258, Phe317 and His396 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(16) The invention relates to an isolated polypeptide which comprises a functional kinase domain comprising the amino acid sequence of the native human Abl kinase domain or an essentially similar sequence thereof in which at least one amino acid selected from Leu248, Glu252, Gly250, Glu355, Glu359, His396, Ser417, Glu459 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(17) The invention relates to an isolated polypeptide which comprises a functional kinase domain comprising the amino acid sequence of the native human Abl kinase domain or an essentially similar sequence thereof in which at least one amino acid selected from Leu248, Gly250, Glu252, Glu355, Glu359, His396, Ser417, Glu459, is replaced by another amino acid and in which optionally at least one other amino acid selected from Met244, Gly250, Tyr253, Val256, Glu258, Phe311, Ile313, Phe317, Met318, Met351, Ile360, His361, Leu370, Asp381, Phe382, His396 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(18) The invention relates to an isolated polypeptide which comprises a functional kinase domain comprising the amino acid sequence of the native human Abl kinase domain or an essentially similar sequence thereof in which at least one amino acid selected from Met244, Leu248, GIy250, Glu252, Tyr253, Val256, Glu258, Phe311, Ile313, Phe317, Met318, Met351, Glu355, Glu359, Ile360, His361, Leu370, Asp381, Phe382, His396, Ser417, Glu459 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(19) The invention relates to an isolated polypeptide which comprises a functional kinase domain comprising the amino acid sequence of the native human Abl kinase domain or an essentially similar sequence thereof in which at least one amino acid selected from Met244, Leu248, Gly250, Glu252, Tyr253, Val256, Glu258, Phe311, Ile313, Phe317, Met318, Glu355, Glu359, Ile360, His361, Leu370, Asp381, Phe382, His396, Ser417, Glu459 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(20) The invention relates to an isolated polypeptide which comprises a functional kinase domain comprising the amino acid sequence of the native human Abl kinase domain or an essentially similar sequence thereof in which at least one amino acid selected from Met244, Leu248, Gly250, Glu252, Tyr253, Glu258, Phe311, Phe317, Met351, Glu355, Glu359, His396, Ser417, Glu459 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(21) The invention relates to an isolated polypeptide which comprises a functional kinase domain comprising the amino acid sequence of the native human Abl kinase domain or an essentially similar sequence thereof in which at least one amino acid selected from Met244, Leu248, Gly250, Glu252, Tyr253, Glu258, Phe311, Phe317, Glu355, Glu359, His396, Ser417, Glu459 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(22) A preferred embodiment of the invention relates to an isolated polypeptide according to any one of the preceding paragraphs (1) - (21), wherein in the amino acid sequence of the native human Abl kinase domain or an essentially similar sequence thereof a single amino acid is replaced by another amino acid.
(23) In a very preferred embodiment the invention relates to an isolated polypeptide which comprises a functional kinase domain comprising the amino acid sequence of the native human Abl kinase domain or an essentially similar sequence thereof that contains at least one amino acid mutation selected from Leu248Val, Gly250Al, Glu252His, Glu355Gly, Glu359Val, His396Arg, Ser417Tyr and Glu459Lys, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(24) In a preferred embodiment the invention relates to an isolated polypeptide according to any one of the preceding paragraph (23), wherein the amino acid sequence of the native human Abl kinase domain may contain at least one additional amino acid mutation selected from Met244Val, Gly250Glu, Tyr253His, Tyr253Phe, Glu258Gly, Phe311 Leu, Phe317Leu, Met351Thr, His396Pro and Phe486Ser
(25) In a preferred embodiment the invention relates to an isolated polypeptide according to any one of the preceding paragraphs (23), wherein the amino acid sequence of the native human Abl kinase domain may contain at least one additional amino acid mutation selected from Met244Val, Gly250Glu, Tyr253His, Tyr253Phe, Glu258Gly, Phe311Leu, Phe317Leu, His396Pro and Phe486Ser
(26) In a second preferred embodiment the invention relates to an isolated polypeptide which comprises a functional kinase domain comprising the amino acid sequence of the native human Abl kinase domain or an essentially similar sequence thereof that contains at least one amino acid mutation selected from Met244Val, Leu248Val, Gly250Glu, Gly250Al, Glu252His, Tyr253His, Tyr253Phe, Glu258Gly, Phe311Leu, Phe317Leu, Met351Thr, Glu355Gly, Glu359Val, His396Pro, His396Arg, Ser417Tyr, Glu459Lys and Phe486Ser, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(27) In a very preferred embodiment the invention relates to an isolated polypeptide which comprises a functional kinase domain comprising the amino acid sequence of the native human Abl kinase domain or an essentially similar sequence thereof that contains at least one amino acid mutation selected from Met244Val, Leu248Val, Gly250Glu, Gly250Al, Glu252His, Tyr253His, Tyr253Phe, Glu258Gly, Phe311Leu, Phe317Leu, Glu355Gly, Glu359Val, His396Pro, His396Arg, Ser417Tyr, Glu459Lys and Phe486Ser, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(28) In a very preferred embodiment the invention relates to an isolated polypeptide which comprises a functional kinase domain comprising the amino acid sequence of the native human Abl kinase domain or an essentially similar sequence thereof that contains at least one amino acid mutation selected from Met244Val, Gly250Glu, Tyr253His, Tyr253Phe, Glu258Gly, Phe311Leu, Phe317Leu, Met351Thr, His396Pro and Phe486Ser, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(29) The invention further relates especially to an isolated polypeptide which comprises a functional kinase domain comprising the amino acid sequence of the native human Abl kinase domain or an essentially similar sequence thereof that contains at least one amino acid mutation selected from Met244Val, Gly250Glu, Tyr253His, Tyr253Phe, Glu258Gly, Phe311Leu, Phe317Leu, His396Pro and Phe486Ser, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(30) The invention further relates very especially to an isolated polypeptide which comprises a functional kinase domain comprising the amino acid sequence of the native human Abl kinase domain or an essentially similar sequence thereof that contains at least one amino acid mutation selected from Gly250Glu, Tyr253His, Tyr253Phe, Glu258Gly, Phe317Leu and His396Pro, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(31) Similarly, the invention relates especially to an isolated polypeptide which comprises a functional kinase domain comprising the amino acid sequence of the native human Abl kinase domain or an essentially similar sequence thereof that contains the amino acid mutation Met351Thr, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(32) Similarly, the invention relates especially to an isolated polypeptide which comprises a functional kinase domain comprising the amino acid sequence of the native human Abl kinase domain or an essentially similar sequence thereof that contains the amino acid mutation Met244Val, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(33) Similarly, the invention relates especially to an isolated polypeptide which comprises a functional kinase domain comprising the amino acid sequence of the native human Abl kinase domain or an essentially similar sequence thereof that contains the amino acid mutation Gly250Glu, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(34) Similarly, the invention relates especially to an isolated polypeptide which comprises a functional kinase domain comprising the amino acid sequence of the native human Abl kinase domain or an essentially similar sequence thereof that contains the amino acid mutation Tyr253His, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(35) Similarly, the invention relates especially to an isolated polypeptide which comprises a functional kinase domain comprising the amino acid sequence of the native human Abl kinase domain or an essentially similar sequence thereof that contains the amino acid mutation Tyr253Phe, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(36) The invention relates also especially to an isolated polypeptide which comprises a functional kinase domain comprising the amino acid sequence of the native human Abl kinase domain or an essentially similar sequence thereof that contains the amino acid mutation Glu25BGly, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(37) The invention relates also especially to an isolated polypeptide which comprises a functional kinase domain comprising the amino acid sequence of the native human Abl kinase domain or an essentially similar sequence thereof that contains the amino acid mutation Phe311 Leu, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(38) The invention relates also especially to an isolated polypeptide which comprises a functional kinase domain comprising the amino acid sequence of the native human Abl kinase domain or an essentially similar sequence thereof that contains the amino acid mutation Phe317Leu, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(39) Similarly, the invention relates especially to an isolated polypeptide which comprises a functional kinase domain comprising the amino acid sequence of the native human Abl kinase domain or an essentially similar sequence thereof that contains the amino acid mutation His396Pro, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(40) The invention relates also especially to an isolated polypeptide which comprises a functional kinase domain comprising the amino acid sequence of the native human Abl kinase domain or an essentially similar sequence thereof that contains the amino acid mutation Phe486Ser, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(41) Similarly, the invention relates especially to an isolated polypeptide which comprises a functional kinase domain comprising the amino acid sequence of the native human Abl kinase domain or an essentially similar sequence thereof that contains the amino acid mutation Leu248Val, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(42) Similarly, the invention relates especially to an isolated polypeptide which comprises a functional kinase domain comprising the amino acid sequence of the native human Abl kinase domain or an essentially similar sequence thereof that contains the amino acid mutation Gly250Ala, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(43) Similarly, the invention relates especially to an isolated polypeptide which comprises a functional kinase domain comprising the amino acid sequence of the native human Abl kinase domain or an essentially similar sequence thereof that contains the amino acid mutation Glu252His, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(44) Similarly, the invention relates especially to an isolated polypeptide which comprises a functional kinase domain comprising the amino acid sequence of the native human Abl kinase domain or an essentially similar sequence thereof that contains the amino acid mutation Glu355Gly, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(45) Similarly, the invention relates especially to an isolated polypeptide which comprises a functional kinase domain comprising the amino acid sequence of the native human Abl kinase domain or an essentially similar sequence thereof that contains the amino acid mutation Glu359Val, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(46) Similarly, the invention relates especially to an isolated polypeptide which comprises a functional kinase domain comprising the amino acid sequence of the native human Abl kinase domain or an essentially similar sequence thereof that contains the amino acid mutation His396Arg, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(47) Similarly, the invention relates especially to an isolated polypeptide which comprises a functional kinase domain comprising the amino acid sequence of the native human Abl kinase domain or an essentially similar sequence thereof that contains the amino acid mutation Ser417Tyr, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(48) Similarly, the invention relates especially to an isolated polypeptide which comprises a functional kinase domain comprising the amino acid sequence of the native human Abl kinase domain or an essentially similar sequence thereof that contains the amino acid mutation Glu459Lys, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(49) In another embodiment the invention relates to an isolated polypeptide according to any one of the preceding paragraphs (31) - (48), wherein the amino acid sequence of the native human Abl kinase domain may contain at least one additional amino acid mutation selected from Met244Val, Leu248Val, Gly250Glu, Gly250Al, Glu252His, Tyr253His, Tyr253Phe, Glu258Gly, Phe311Leu, Phe317Leu, Met351Thr, Glu355Gly, Glu359Val, His396Pro, His396Arg, Ser417Tyr, Glu459Lys and Phe486Ser.
(50) In a preferred embodiment the invention relates to an isolated polypeptide according to any one of the preceding paragraphs (1) - (49), wherein the amino acid sequence of the native human Abl kinase domain consists of amino acids 229-500 of SEQ ID NO:2.
(51) In another preferred embodiment the invention relates to an isolated polypeptide according to any one of the preceding paragraphs (1) - (50), said isolated polypeptide being a Bcr-Abl tyrosine kinase.
(52) In yet another preferred embodiment the invention relates to the use of an isolated polypeptide of any one of the preceding paragraphs (1) - (51) to screen for compounds which inhibit the tyrosine kinase activity of said polypeptide.
(53) The invention also relates to an isolated nucleic acid molecule comprising a nucleotide sequence that encodes a polypeptide according to any one of the preceding paragraphs (1) - (51).
(54) The invention further relates to the use of a nucleic acid molecule of the preceding paragraph (53) in the production of a polypeptide of any one of the preceding paragraphs (1) - (51) for use in screening for compounds which inhibit the tyrosine kinase activity of said polypeptide.
(55) The invention also relates to a recombinant vector comprising a nucleic acid molecule according to the preceding paragraph (53).
(56) The invention further relates especially to a recombinant vector according to the preceding paragraph (55), which is a recombinant expression vector.
(57) The invention also relates to a host cell comprising a recombinant vector according to the preceding paragraph (55) or (56).

Preferably the invention relates to an isolated polypeptide which comprises a functional kinase domain comprising the amino acid sequence of the native human Abl kinase domain in which at least one amino acid is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

A preferred salt of STI571 is the methane sulfonate salt described in WO 99/03854.

Screening for compounds which inhibit the tyrosine kinase activity of the polypeptides of the invention may be done for example by using an isolated polypeptide of the invention in any *in vitro* tyrosine kinase phosphorylation assay known in the art and determining the potential of a compound to inhibit the tyrosine kinase activity of a polypeptide of the invention in such an assay.

High-throughput screening assays known in the art may be used to screen large compound libraries for compounds which inhibit the tyrosine kinase activity of the polypeptides of the invention.

Besides the random screening of large compound libraries, the polypeptides of the present invention may also be used in the following screening approach: The 3-dimensional structure of a polypeptide of the invention is determined by e.g. X-ray crystallography. The atomic coordinates of a polypeptide of the invention are then used to design a potential inhibitor. Said potential inhibitor is then synthesized and tested for its ability to inhibit the tyrosine kinase activity of the polypeptide of the invention in any *in vitro* tyrosine kinase phosphorylation assay.

### Examples:

The following Examples serve to illustrate the invention without limiting its scope.

### Example 1:

Numerous mutations in the Abl kinase domain were generated based on three models of STI571 bound to the Abl kinase domain, and these mutant kinases were assessed for sensitivity to STI571.

A variety of point mutations were generated based on a model of the Abl kinase domain cocrytallized with a STI571-related 2-phenylaminopyrimidine Abl-specific inhibitor [Schindler T. et al., Science 289, 1938-42 (2000)]. The amino acids identified by the Abl crystal structure as potential contact sites for STI571 include hydrogen bonds with T315, M290, E286, K271 and the peptide backbone at D381 and M318, as well as hydrophobic interactions with 1313, F382, V256, Y253 and L370. Mutations were generated to eliminate the potential for hydrogen bonding or hydrophobic interactions between the Abl kinase and STI571 [The Abl kinase domain consisting of c-Abl amino acids 220 to 458 was subcloned into the Bam HI site of pGEX KG (Pharmacia). A Hemagglutin (12CA5) antibody recognition tag was inserted at the 5' end of the Abl kinase domain sequence both as an Abl phosphorylation site and for detection of protein expression. All mutations within the kinase domain were constructed using polymerase chain reaction amplification of the Abl kinase pGEX KG plasmid with primers containing appropriate point mutations.].

As M318 and D381 are predicted to form hydrogen bonds with STI571 via the peptide backbone, mutations at these sites are irrelevant. The IC₅₀ values of the mutations are summarized in Table 1 [GST fusion proteins of the Abl kinase domain mutations as well as wild type Abl kinase were generated by inducing exponentially-growing transformed DH5α bacteria with 1 mM isopropylphenylthiogalactoside (IPTG). The cells were lysed via sonication in MT PBS (150 mM NaCl, 16 mM Na₂HPO₄, 4 mM Na₂H₂PO₄, pH 7.3) containing 1% Triton-X 100, 10 µg/ml aprotinin, 1 mM sodium vanadate, 1 mM phenylmethylsulfonyl fluoride and 10 µM β-mercaptoethanol. The GST-Abl kinase mutants were purified from the lysate by binding to glutathione sepharose overnight at 4 °C. Bound proteins were washed twice with 0.5 M LiCl, twice with PBS (Phosphate Buffered Saline pH 7.5) and once with Abl kinase wash buffer (20 mM Tris pH 7.5, 10 mM MgCl₂). Bound protein concentrations were determined by SDS PAGE followed by Coomassie Blue staining. All Abl kinase proteins and mutations were expressed and purified in this manner. 500 ng of bound protein was used in each kinase reaction. Kinase reactions were performed in 30 µl of Abl kinase buffer (20 mM Tris pH 7.5, 10 mM MgCl₂, 10 µM Sodium Vanadate, 1 µM DTT, 1% Dimethyl Sulfoxide (DMSO)). STI571 was dissolved in 3% DMSO prior to addition to the kinase reaction. The Abl kinase mutations were incubated with concentrations of STI571 ranging from 0 µM to 1 µM for 10 minutes, after which 10 µCi of γ ³²P ATP (100 µM total ATP) was added and the kinase reaction allowed to proceed for 30 minutes. The reactions were terminated by boiling in SDS loading dye and samples were analyzed by SDS PAGE (Equal protein loading was demonstrated by immunoblots using the anti-Abl kinase domain antibody Ab-2 (Santa Cruz)). Abl autophosphorylation signal intensity was quantitated with a phosphorimager (Molecular Dynamics) and IC₅₀ values were determined]. Consistent with what has been reported using similar kinase assays, the IC₅₀ value for wild type Abl kinase was 0.025 µM. K271 R, E286L, M290A and I313G yielded kinase inactive mutations. Mutation of L370G did not change the sensitivity to STI571. In contrast, T315V demonstrated a decreased sensitivity to STI571. The IC₅₀ value of this mutation averaged to 0.30 µM, approximately ten-fold higher than that of wild type Abl kinase. The decreased sensitivity of this mutation to STI571 relative to wild type Abl is consistent with predictions from the crystal structure that illustrates a critical hydrogen bond between the secondary amino group of the inhibitor and the side chain of T315.

Prior to the availability of the crystal structure, mutations were generated based on computer modeling of STI571 bound to Abl, based on published kinase domain structures. These mutations were analyzed to determine whether the activity of these mutations could be fit to the published Abl kinase domain structure. For example, a computer model of Abl based on the crystal structure of the Src family member Lck was initially analyzed [Yamaguchi H. and Hendrickson W.A., Nature 384, 484 (1996)]. Mutations of the predicted contact points from the Lck-based model were made to the corresponding residues in either Src or Fms (Table 2). The majority of these mutations were either kinase inactive or did not display any change in sensitivity to STI571. These results support predictions from the crystal structure that suggests that topological differences rather than amino acid sequence differences in the ATP binding pocket of the Abl kinase and the Src family kinases are responsible for the specificity of STI571. Interestingly, mutation of A380T raised the IC₅₀ for STI571 binding ten-fold over that of wild type Abl kinase. While this residue is not a predicted contact point in the Schindler et al. model [Schindler T. et al., Science 289, 1938-42 (2000)], the adjacent residue D381 forms a critical hydrogen bond with STI571. Because mutation of A380 to the smaller glycine did not alter the sensitivity to STI571, it is likely that mutation to a slightly larger residue either introduces steric effects or small conformational changes in the STI571 binding surface that can account for the decrease in sensitivity of the A380T mutation. Thus, all of the predictions from the Lck kinase can be explained by the Abl crystal structure.

A third model of STI571 binding to the Abl kinase is extrapolated from computer modeling of fibroblast growth factor receptor (FGFR) [Mohammadi M. et al., Science 276, 955 (1997)]. In this model STI571 binds in reverse orientation relative to that seen in the Schindler et al. model. Additionally, several new potential contact points are identified including E258, M318 and L248. This binding mode also predicts STI571 contacts V256 and L370 which are predicted contact sites in the crystal structure. Mutations of these residues were examined for their sensitivity to STI571 (Table 3). As with many of the previous mutations, several of these were kinase inactive. Interestingly, the E258G mutation demonstrated an IC₅₀ value of 0.18 µM, eight-fold higher than that of wild type Abl kinase. The decrease in sensitivity of the mutation to STI571 suggests that the hydrogen bonding capabilities of E258 may be critical to interactions between STI571 and the Abl kinase.

**Table 1. Mutations were made to residues lacking either hydrogen bond-forming capabilities or hydrophobicity. STI571 was predicted to form hydrogen bonds with the peptide backbone at M318 and D381, therefore mutations of these residues were not made. Many of the mutations resulted in a kinase inactive Abl. L370G did not change the sensitivity to STI571. Mutation of T315V decreased the sensitivity to STI571 ten-fold relative to wild type Abl kinase.**

| **Potential Contact Sites** | **Mutations** | **Sensitivity to STI571** |
|---|---|---|
| wild-type (wt) | - | IC₅₀ = 0.025 µM |
| K271 | R | kinase inactive |
| E286 | L | kinase inactive |
| M290 | A | kinase inactive |
| 1313 | G | kinase inactive |
| T315 | V | IC₅₀ = 0.30 µM |
| M318 | - | - |
| L370 | G | same as wt |
| D381 | - | - |

**Table 2. Abl kinase domain mutations based on an Lck computer model of inhibitor binding. The structure of the inactive form of the Abl kinase was modeled using information from the crystal structure of inhibitor-bound Lck (from Kinetix Pharmaceuticals). Mutations were made of the predicted contact points of Abl with STI571 to the corresponding residues in either Src or fms. Most of the mutations resulted in either no change in sensitivity to STI571 or a kinase inactive Abl. Mutation of A380T decreased the sensitivity to STI571 ten-fold relative to wild type Abl kinase. This is likely due to steric effects or conformational changes in the STI571 binding surface of the mutated Abl.**

| **Potential Contact Sites** | **Mutations** | **Sensitivity to STI571** |
|---|---|---|
| wild-type (wt) | - | IC₅₀ = 0.025 µM |
| L248 | A | kinase inactive |
| Y320 | K | same as wt |
| N322 | S | same as wt |
| E373 | N | same as wt |
| H375 | L | kinase inactive |
| A380 | C | same as wt |
| A380 | T | IC₅₀ = 0.34 µM |
| A380 | L | kinase inactive |

**Table 3. Abl kinase domain mutations based on a Fibroblast Growth Factor Receptor model of inhibitor binding. Mutations were made of the predicted contact points of Abl with STI571 that would eliminate hydrogen bonding or large hydrophobic side chains. Many of the mutations yielded a kinase inactive Abl. Mutation of E258G decreased the sensitivity to STI571 eight-fold relative to wild type Abl kinase.**

| **Potential Contact Sites** | **Mutations** | **Sensitivity to STI571** |
|---|---|---|
| wild-type (wt) | - | IC₅₀ = 0.025 µM |
| L248 | A | kinase inactive |
| V256 | G | kinase inactive |
| E258 | G | IC₅₀ = 0.18 µM |
| M318 | A | kinase inactive |
| L370 | G | same as wt |

Further structural studies revealed that also the residues Ile360 and His361 of the Abl kinase domain are involved in the binding of STI571. Amino acid mutations at these positions therefore have the potential to confer STI571-resistance.

### Example 2:

Clinical samples of six patients prior to therapy with STI571 and at the time of relapse under therapy with the drug were analyzed for the presence of mutations in the ATP-binding pocket and the activation loop of the Abl kinase domain. Of the patients analyzed, two suffered from advanced stage CML (lymphoid blast crisis, one patient; accelerated phase CML progressing to blast crisis at the time of relapse, one patient), three patients from Ph+ ALL, and one patient from biphenotypic Ph+ acute leukemia (see Table 4).

**Table 4. Clinical data**

| **Pat. No.** | **Disease** | **Bcr-Abl- transcript** | **Previous therapy** | **Additional cytogenetic abnormalities*** | **Duration of therapy** | **Max. response, duration** |
|---|---|---|---|---|---|---|
| | | | **(y/n)** | **(y/n)** | **(weeks)** | **(weeks)** |
| 1 | Ph+ ALL | p210 | y | n | 28** | CHR (15) ; PCR |
| 2 | CML AP/BC myeloid | p210 | y | n | 9 | RCP (6)*** |
| 3 | CML BC lymphoid | p210 | y | n | 7 | No^{#} |
| 4 | Ph+ AL biph. | p190 | y | y | 15 | No^{##} |
| 5 | Ph+ ALL | p190 | y | n | 30 | CHR (24); PCR |
| 6 | Ph+ ALL | p190 | y | n | 8⁺ | CHR^{§} (4) |
| | | | | | 6⁺⁺ | NLE^{§§} (2) |

| | | | | | | |
|---|---|---|---|---|---|---|
| Ph+: Philadelphia-chromosome-positive; ALL: Acute lymphoblastic leukemia; CML: Chronic myeloid leukemia; AP: Accelerated phase, based on percentage of blasts in bone marrow >15% but <30%; BC: Blast crisis, based on percentage of blasts in bone marrow >30%; CHR: Complete hematological remission, based on all of the following criteria: Blast count <5% in bone marrow, no circulating peripheral blood blasts, absolute neutrophil count >1.5x10⁹/L, platelet count >100x10⁹/L, no evidence of extramedullary involvement; PCR: Partial cytogenetic remission, based on the finding of 5/100 phchromosome-positive metaphases; RCP: Return to chronic phase, based on all of the following criteria: Percentage of blasts in blood or bone marrow <15%, percentage of blasts plus promyelocytes in peripheral blood or bone marrow <30%, peripheral blood basophils <20%; Biph.: Biphenotypic disease due to expression of both myeloid, and lymphoid surface markers. *Prior to therapy with STI571 **Continuing with STI571, 800 mg per day due to relapse of Ph+ ALL ***Fulminant relapse to CML blast crisis receiving STI571 600 mg per day ^{#}Pancytopenia with persistence of blastoid cells in peripheral blood and >30% blastoid cells in bone marrow ^{##}No circulating peripheral blood blasts and absolute neutrophil count >1.0x10⁹/L for a period of 10 weeks, but persistent blast count >5% in bone marrow ⁺STI571, 600 mg per day ⁺⁺Switch to STI571, 800 mg per day due to relapse (White cell count 360x10⁹/L, 85% blastoid cells) receiving STI571 600 mg per day ^{§}Bone marrow not evaluated ^{§§}NLE: No leukemic evidence in peripheral blood, based on the following findings: No circulating peripheral blood blasts, absolute neutrophil count >1.0x10⁹/L, platelet count >20x10⁹/L, bone marrow has not been evaluated. | | | | | | |

Total RNA from purified peripheral blood and/or bone marrow cells was extracted using RNAClean (Hybaid GmbH, Heidelberg, Germany). 10 ng RNA per clinical sample, 10 pg K562 total RNA as positive-control and a reaction without template as negative-control were subjected to reverse transcriptase-polymerase chain reaction (RT-PCR) using a 3' Abl-specific primer (5'-GCCAGGCTCTCGGGTGCAGTCC-3') and a 5' Abl-specific primer (5'-GCGCAACAAGCCCACTGTCTATGG-3'). AMV reverse transcriptase was used for first strand synthesis, Expand high fidelity (Taq DNA polymerase and a proofreading polymerase; Roche Molecular Biochemicals, Mannheim, Germany) for amplification. Human β-action served as control using forward primer 5'-CCAAGGCCAACCGCGAGAAGATGAC-3' and reverse primer 5'-AGGGTACATGGTGGTGCCGCCAGAC-3' (Roche Molecular Biochemicals, Mannheim, Germany). The amplified 579bp fragment was sequenced with an ABI 3700 sequencer (PE Biosystems, Foster City, CA, USA) using two 3' Abl-specific primers (5'-GCCAGGCTCTCGGGTGCAGTCC-3' and 5'-CAAGTTCCCCATCAAATG-3') and two different 5' Abl-specific primers, (5'-GCGCAACAAGCCCACTGTCTATGG-3' and 5'-ATGGAGGTGGAAGAGTTC-3'), respectively. Sequence analysis was performed using Lasergene software (DNA*, Madison, USA). Two to nine RT-PCR reactions per patient and time point were performed and analyzed independently showing a sequence identity of 100% at each patient and time point. Overall sequence identity of all probes excluding the six single point mutations found at the time of STI571-refractory disease to one another and to the sequence of human c-Abl (Gl:28236) was 100%.

**Table 5. Sequence analysis**

| | **Prior to STI571** | | | **At the time of resistence to STI571** | | | | |
|---|---|---|---|---|---|---|---|---|
| **Pat. No.** | | | | | | | | |
| | **No. of RT-PCR- reactions** | **No. of identical sequences** | **No. of mutations found** | **No. of RT-PCR- reactions** | **No. of identical sequences** | **No. of mutations found** | **Position of mutation^{#}** | |
| | | | | | | | **Nucleotide** | **Amino acid** |
| **1** | 9⁺ | 9⁺ | 0 | 6 | 6 | 1 | 911 A→T | 255 Glu→Val |
| **2** | 2 | 2 | | 2 | 2 | 0 | - | - |
| **3** | 4 | 4 | 0 | 2 | 2 | 1 | 910 G→A | 255 Glu→Lys |
| **4** | 2 | 2 | 0 | 2 | 2 | 1 | 904 T→C | 253 Tyr→His |
| **5** | 4 | 4 | 0 | 2 | 2 | 1 | 1334 A→C | 396 His→Pro |
| **6** | 2 | 2 | 0 | 2* | 2* | 1 | 1091 C→T | 315 Thr→Ile |
| | | | | 2** | 2** | 1 | 1091 C→T | 315 Thr→Ile |

| | | | | | | | | |
|---|---|---|---|---|---|---|---|---|
| *1^{st} relapse receiving STI571 600 mg per day **2^{nd} relapse receiving STI571 800 mg per day ^{#}Nucleotides / amino acid residues are numbered from the first nucleotide / amino acid of human c-Abl (Accession number: Gl:28236) ⁺Analysis of different time points | | | | | | | | |

Of six patients, sequence analysis revealed the presence of a single point mutation in clinical samples of five patients. All five mutations result in an amino acid exchange within the ATP-binding site (Patients 1, 3, 4 and 6, see Table 5) or activation loop (Patient 5) of Abl kinase domain and were present only in the samples obtained at the time of refractory disease. In one patient (No. 6, see Table 5), we were able to detect a point mutation of human c-Abl kinase domain at nucleotide 1091 C→T (numbered from the first nucleotide of human c-Abl, Gl:28236) leading to a Thr→Ile change at position 315 of the ATP-binding site (numbered from the first amino acid of human c-Abl, Gl:28236). Interestingly, this patient experienced a second response after increasing the daily dose of STI571 from 600 to 800mg despite the presence of T315I at the time of refractory disease receiving the lower dose (see Table 4 + 5). Having in mind the presence of Thr315 being a key requirement for STI571 to bind and thus inhibit Abl [Schindler T. et al., Science 289, 1938-42 (2000)] this observation should require the presence of unmutated Bcr-Abl contributing to growth of the malignant clone. This seems to be true: Single nucleotide polymorphism (SNP) analysis of all the four samples obtained at the time of refractory disease showed evidence for a SNP at position 1091 of c-Abl, visible in the chromatographs as C-background behind the T-signal and, comparing the samples obtained at the time of refractoriness to 600 mg and those obtained at the time of refractoriness to 800mg, this heterogeneity significantly decreases in favour of the mutated transcript. This finding strongly suggests, that T315I, when present in the majority of Bcr-Abl molecules, abrogates the biologic activity of STI571. A tyrosine at position 253, which is changed to histidine in patient no. 4 (see Table 5), has been shown to form a hydrogene bond with asparagine 322, holding in place a folded loop between two β strands, increasing the surface complementarity with STI571 and, furthermore to hold a van der Waals interaction with the aromatic rings of STI571 [Schindler T. et al., Science 289, 1938-42 (2000)].

Both point mutations seen in patients 1 and 3, respectively (see Table 5) result in an exchange of glutamic acid (hydrophilic, negatively charged) at position 255 of ATP-binding site to valine (hydrophobic) in patient 1 and lysine (hydrophilic, positively charged) in patient 3. Considering the interaction of neighbouring valine 256 with one of the aromatic rings of STI571 [Schindler T. et al., Science 289, 1938-42 (2000)], this change may also lead to a conformational change impairing the binding of the drug.

The mutation found in patient 5, a change of histidine at position 396 to proline, is located within the activation loop. This region of Abl does not interact directly with STI571, except for the anchor region, located NH₂-terminal of His 396, but is in its inactive (closed) conformation a prerequisite for specific binding of STI571 to Abl [Schindler T. et al., Science 289, 1938-42 (2000)]. One may speculate that this mutation may stabilize the activation loop in an open conformation, which has been shown to be less susceptible to inhibition by STI571 after stabilizing the open conformation by phosphorylation of Tyr393, the major site of phosphorylation in Abl [Schindler T. et al., Science 289, 1938-42 (2000)].

In summary, our analysis of six patients shows that in cases of STI571 refractory Ph+ leukemia, mutations within the ATP-binding site or activation loop occur frequently. Thus, Bcr-Abl dependent proliferation of the malignant clone may be restored by impairing the binding of STI571 to Bcr-Abl without compromising Bcr-Abl kinase-activity.

### Example 3:

A PCR strategy was used to amplify the ATP binding region of Bcr-Abl cDNA and the entire region was sequenced in both directions. Bcr-Abl mutations were found in 4/6 patients on STI571 for blast crisis CML (2 myeloid, 2 lymphoid BC), accelerated phase (1) or second chronic phase CML (1) who had developed haematological resistance to STI571. One patient had the mutation Thr315Ile. Two patients had mutations at position 250, which substituted glycine for glutamic acid. One patient had a mutation at amino acid 253, which substituted tyrosine for histidine. Amino acid 250 does not form a hydrogen bond with STI571, as does amino acid 315, nor is it involved in van der Waals interaction with the inhibitor, as is amino acid 253. Where samples were available (3 cases) we confirmed that the mutation was not present prior to STI571 therapy, nor was it present in the normal Abl allele. We also sequenced the ATP binding region of Bcr-Abl in 8 patients with advanced phase (5) or chronic phase (3) CML who had achieved and maintained haematological responses but only had minor or no cytogenetic response on STI571. None of these patients had evidence of mutations after 6-9 months of ST1571 therapy. We postulate that several point mutations in Bcr-Abl emerge in patients with advanced phase CML which are likely to totally or partially abrogate STI571 binding to Bcr-Abl.

### Example 4:

An RT-PCR strategy was used to amplify and sequence the Abl kinase domain of Bcr-Abl in 28 patients. We selected STI571-refractory and STI571-resistant patients from 253 patients enrolled in expanded access studies at 5 Australian centres. The aim was to determine the frequency and timing of acquired mutations within carefully defined clinical groups, determine their distribution within the Bcr-Abl kinase domain and identify any association between specific mutations and clinical features.

### Study Design

ST1571-resistant patients (n=18) were defined as those with a loss of complete haematological remission which had been present for at least 3 months or evolution to acute phase CML or relapsed Ph+ ALL. STI571-refractory patients (n=10) were those who failed to achieve a major cytogenetic response after at least 6 months of therapy.

Extraction of RNA from blood, reverse transcription and DNA sequencing procedures have been previously described [Branford S. et al., Br. J. Haematol. 107, 587-599 (1999)]. A long PCR method [Branford S. et al., Br. J. Haematol. 109, 635-637 (2000)] was used to amplify the Abl kinase domain of Bcr-Abl with forward primer BcrF (5' tgaccaactcgtgtgtgaaactc) and reverse primer AblKinaseR (5' tccacttcgtctgagatactggatt). A second stage PCR used forward primer AbikinaseF (5' cgcaacaagcccactgtct) and reverse primer AblkinaseR. The entire kinase domain was sequenced, an area including 863 bases (GenBank accession number M14752).

### Results

### STI571-resistant patients:

Twelve of 18 STI571-resistant patients had mutations in the ATP binding region of Bcr-Abl (Table 6). In 9 cases where samples were available, we confirmed that the mutation was not present before starting STI571 therapy, nor was it present in 4 cases tested at 3-9 months, which in each case was before the onset of resistance. Three of the 6 resistant patients without Bcr-Abl mutations had evidence of clonal evolution at the time of relapse including an additional Philadelphia (Ph) chromosome in 2 cases. One patient had both a mutation and an extra Ph chromosome at time of relapse.

The 6 mutations identified are T315I (n=3 patients), Y253H (n=1), F317L (n=1), E255K (n=4), G250E (n=2) and M351T (n=1).

The 18 STI571-resistant patients could be subdivided into those who relapsed into blast crisis or Ph+ ALL (n=8), those relapsing into accelerated phase (n=6) and those with evidence of loss of hematological remission who remained in chronic phase (n=4). All 3 groups included patients with mutations. Six of the 8 patients who relapsed directly into blast crisis/ALL had mutations. In 3 of these the T315I mutation was present. Two of the 6 patients relapsing into accelerated phase had mutations. The remaining 4 patients relapsing into chronic phase all had mutations.

### STI571-refractory patients:

Only 1 of 10 refractory patients had a mutation (Table 6). There was no evidence of the mutation pre-STI571 therapy or 3 months after starting treatment. The mutant clone mixed with wild-type Bcr-Abl emerged at 8 months and persisted in a mixed pattern until the mutant clone became predominant at 11 months. To date there has been no clinical evidence of resistance in this patient.

**Table 6. Clinical course and mutation analysis of patients treated with STI571**

| Patient ID | Age /Sex | Disease status at start of STI571* | Duration of STI571 Treatment | Best Response to STI571* | Response at Time of Mutation Analysis* | Time of Mutation Anlaysis † | Mutation Result | Nucleotide Substitution (GenBank no.M14752) |
|---|---|---|---|---|---|---|---|---|
| Emerging Resistance | | | | | | | | |
| 01 | 61 F | 3(3^{rd}) | 9m | | 1b | Pre Study | NM | |
| | | | | 7 | 7 | 3m | NM | |
| | | | | | 7 | 6m | NM | |
| | | | | | 1b | 9m | T315I | G to C nt 944 |
| | | | | | | | | |
| 02 | 75 F | 1a | 4.5m | | 1a | Pre study | | |
| | | | | 4 | 1a | 4.5m | T315I | G to C nt 944 |
| 03 | 62 M | 1b | 2m | | 1b | Pre study | | |
| | | | | 4 | 1b | 2m | T315I | G to C nt 944 |
| 04§ | 59 F | 1c | 3m | | 1c | Pre Study | NM | |
| | | | | 7 | 1c | 5m | Y253H | T to C nt 757 |
| | | | | | 1c | 5.5m | Y253H | T to C nt 757 |
| 05 | 40 M | 3 | 8m | | 3 | Pre Study | NM | |
| | | | | 5 | 5 | 4m | F317L | C to G nt 951 |
| | | | | | 4 | 7m | F317L | C to G nt 951 |
| 06 | 66 M | 1b | 8m‡ | 4 | 1b | 8m | G250E | G to C nt 749 |
| 07 | 54 F | 1a | 10.5m | | 1a | Pre Study | NM | |
| | | | | 5 | 5 | 6m | NM | |
| | | | | | 5 | 9m | NM | |
| | | | | | 5 | 10m | G250E | G to C nt 749 |
| | | | | | 4 | 10.5m | G250E | G to C nt 749 |
| 08 | 41 M | 2 | 6m | 5 | 2 | 6m | E255K | G to A nt 769 |
| 09 | 60 M | 2 | 5m | 5 | 5 | 3m | NM | |
| | | | | | 4 | 6m | E255K | G to A nt 769 |
| 10 | 44 M | 2 | 4.5m | | 2 | Pre Study | NM | |
| | | | | 5 | 4 | 4m | E255K | G to A nt 769 |
| 11§ | 60 F | 1c | 3m | | 1c | Pre Study | NM | |
| | | | | 5 | 1c | 3m | E255K | G to A nt 769 |
| 12 | 52 F | 3 | 9m | | 3 | Pre Study | NM | |
| | | | | 7 | 7 | 6m | NM | |
| | | | | | 7 | 7m | NM | |
| | | | | | 6 | 8m | M351T | T to C nt 1052 |
| | | | | | 2^{P} | 9m | M351T | T to C nt 1052 |
| 13 | 64 M | 3 | 10.5m | 5 | 2^{P} | 10m | NM | |
| 14 | 78 F | 1a | 8m | 5 | 2^{C+P} | 8m | NM | |
| 15 | 47 M | 2^{c} | 9m | 2^{c} | 1^{C} | 7m | NM | |
| 16 | 56 F | 3 | 7m | 5 | 2 | 6m | NM | |
| 17 | 37 M | 1a | 7m | 4 | 1a | 4.5m | NM | |
| 18 | 63 M | 2 | 7m | 4 | 2 | 8m | NM | |

| Refractory | | | | | | | | |
|---|---|---|---|---|---|---|---|---|
| 19 | 55 F | 2^{C} | 6m | 4 | 4^{C} | 3.5m | NM | |
| 20 | 63 M | 2^{C} | 11m | | 2^{C} | Pre Study | NM | |
| | | | | 2^{c} | 2^{C} | 3m | NM | |
| | | | | | 2^{C} | 8m | M351T | T to C nt 1052 |
| | | | | | 2^{C} | 9m | M351T | T to C nt 1052 |
| | | | | | 2^{C} | 10m | M351T | T to C nt 1052 |
| | | | | | 2^{C} | 11m | M351T | T to C nt 1052 |
| 21 | 62 F | 2 | 10.5m | 5 | 4 | 9m | NM | |
| 22 | 54 M | 2 | 8m | 5 | 5 | 6m | NM | |
| 23 | 61 F | 3 | 6m | 5 | 4 | 5m | NM | |
| 24 | 61 F | 3 | 10.5m | 5 | 4 | 6m | NM | |
| 25 | 47 F | 3 | 10m | 5 | 5 | 6m | NM | |
| 26 | 40 F | 3 | 6m | 5 | 5 | 3m | NM | |
| 27 | 60 M | 3 | 6m | 5 | 5 | 4m | NM | |
| 28 | 42 M | 3 | 7m | 5 | 5 | 6m | NM | |

| | | | | | | | | |
|---|---|---|---|---|---|---|---|---|
| F: female; M: male; m: months. NM Indicates no mutation detected following sequencing of the kinase region of Bcr-Abl in both directions. ^{C}Indicates clonal evolution. ^{P}Indicates double Philadelphia chromosome. *Disease Status/Response: 1 a) myeloid blast crisis b) lymphoid blast crisis c) Ph-positive ALL relapse. 2) Accelerated Phase, 3) Chronic Phase, 4) Partial Response (blasts in blood + bone marrow < 15%), 5) Complete Haematologic Response (WBC<10.0, Plts < 450, no blasts, myelocytes + metamyelocytes < 5%, no promyelocytes, no disease-related symptoms or extramedullary disease), 6) Major Cytogenetic Response, 7) Complete Cytogenetic Response. † Months since treatment start. ‡ Although patient was on study for 8 months, STI571 was alternated monthly with oral arsenic and therefore the patient received only 4 months of STI571 over this period. §Ph-positive ALL patients. | | | | | | | | |

Further studies revealed the presence of two additional mutations, i.e. Met244Val and Phe486Ser, in STI571-resistant/refractory patients suffering from Ph chromosome-positive leukemia.

In another study, CML patients from 12 centers within Australia and New Zealand were tested for mutation analysis. The samples were primarily collected for molecular assessment of BCR-ABL levels and only proceeded to mutation analysis if stored RNA contained a measurable level of BCR-ABL and the control gene level indicated non-degraded RNA. Patients had either received 6 or more months of imatinib therapy or had developed resistance and ceased therapy before 6 months (n=2). Samples from 156 patients were available but 12 could not be tested, 4 due to low levels of BCR-ABL and 8 due to inadequate RNA quality. The remaining 144 patients were grouped according to the disease stage at start of imatinib; 40 in AP, 64 in late-CP defined as ≥12 months since diagnosis and 40 in early-CP defined as <12 months since diagnosis. Sixteen of the early-CP patients had failed previous interferon therapy and 24 had only received hydroxyurea prior to imatinib therapy. Response to imatinib was categorised by the cytogenetic analysis at 6 months as either a major cytogenetic response (MCR) if the number of Philadelphia chromosome positive cells was <35% or no MCR if 35-100%. Imatinib resistance was defined as loss of a complete hematologic remission (CHR) that had been present for at least 3 months, loss of CHR with transformation to accelerated or blastic phase, or loss of an established MCR or a complete cytogenetic response (CCR defined as Philadelphia chromosome negative). 346 RNA samples were analysed as already mentioned herein. Depending on available RNA, patients were tested for mutations at between 1-15 different time-points. The median duration of imatinib therapy was therefore determined from the last time-point of analysis. AP patients had received a median of 9 months of imatinib (range 4 to 24), late-CP 10 months (range 6-24) and early-CP 14 months (range 5-24).

### Imatinib resistance in accelerated phase patients

Fourteen of 40 AP patients developed imatinib resistance, 5 with transformation to blast phase, 8 with recurrence of AP and 1 with loss of CCR. Mutations were detected in 12 of 14 (86%) resistant patients at a median of 8 months of imatinib therapy (range 4-13).

### Imatinib resistance in late chronic phase patients (late-CP)

Thirteen of 64 late-CP patients developed imatinib resistance, 5 with transformation to blast phase, 6 to AP and 2 lost a MCR. Mutations were detected in 11 of 13 (85%) resistant patients at a median of 8 months of imatinib therapy (range 3-18).

### Imatinib resistance in early chronic phase patients (early-CP)

Six of 40 early-CP patients developed resistance, 1 transformed to blast crisis, 2 to AP, 2 lost CCR and 1 lost MCR. No mutations were detected in the resistant patients.

### Duration of CML and the development of mutations

When all the patients in the study who achieved a major cytogenetic response (MCR) within 6 months were studied, those treated >4 years since diagnosis had a 9 times higher incidence of mutations than those treated within 4 years (6 of 22 (27%) versus 2 of 72 (3%)). When all the patients who did not achieve a MCR within 6 months were studied, those treated >4 years since diagnosis had a 2 times higher incidence of mutations than those treated within 4 years (12 of 22 (54%) versus 7 of 28 (25%)). The highest incidence of mutations was in AP patients treated >4 years from diagnosis and who failed to achieve a MCR by 6 months (8 of 12, 67%). In this study 27 of the 144 patients developed mutations. The median duration of CML prior to commencing imatinib therapy of the 27 patients with mutations (5.3 years, range 1.1 to 11) was statistically different to the 117 patients without mutations (1.3 years, range 0.02-17.7) p<0.0001.

### Mutations in the BCR-ABL kinase domain

Table 7 details the 17 different mutations in the BCR-ABL kinase domain detected in 27 patients. These were all point mutations and were located within a sequence of 728 nucleotides involving amino acids 244 to 486. Seven patients had 2-4 mutations and one patient had 2 different mutations at the same nucleotide which both altered the amino acid at position 252 from glutamine to histidine. The mutations, L248V at the N-terminal and S417Y, E459K and F486S at the C-terminal of the kinase domain have not previously been described. The first 3 mutations were all detected in one imatinib resistant patient who also had the G250E mutation.

**Table 7. BCR-ABL kinase domain mutations**

| Mutation | Nucleotide Substitution (GenBank number M14752) | Number of patients with the mutation* |
|---|---|---|
| M244V | 730A>G | 1 |
| L248V | 742C>G | 1 |
| G250E | 749G>A | 3 |
| Q252H | 756G>C | 3 |
| Q252H | 756G>T | 1 |
| Y253F | 758A>T | 2 |
| E255K | 763G>A | 1 |
| E255V | 764A>T | 5 |
| T315I | 944C>T | 2 |
| F317L | 951 C>G | 2 |
| M351T | 1052T>C | 8 |
| E355G | 1064T>C | 3 |
| F359V | 1075T>G | 2 |
| H396R | 1187A>G | 1 |
| S417Y | 1250C>A | 1 |
| E459K | 1375G>A | 1 |
| F486S | 1457T>C | 1 |

| | | |
|---|---|---|
| *7 patients had multiple mutations | | |

### Example 5:

Four molecular and chromosomal mechanisms of resistance were inverstigated in 42 patients (pts.) (21 males, 21 females; median age 60, range 26-70 years) refractory or resistant to STI571 monotherapy (400-800 mg p.o./day) out of a total of 290 pts. treated with STI571 for chronic myeloid leukemia (CML) in chronic phase (CP, n=136), accelerated phase (AP, n=80), myeloid blast crisis (BC, n=73), and lymphoid BC (n=1). Pts. were recruited into six multicenter phase II studies. Prior to STI571 therapy resistant pts. were in myeloid (n=28) or lymphoid BC (n=1), AP (n=9), or CP (n=4). The median duration of therapy was 123 (range 13-741) days. Prior to STI571 and at the time of resistance the expression of Bcr-Abl transcripts was determined in peripheral blood leukocytes by quantitative RT-PCR, the number of genomic Bcr-Abl copies by interphase fluorescence in situ hybridization (IP-FISH), and clonal karyotypic evolution by metaphase cytogenetics. The STI571 binding site of the Bcr-Abl tyrosine kinase domain was sequenced from cDNA derived from resistant blasts. Results: (i) The median level of Bcr-Abl transcripts, expressed as the ratio Bcr-Abl/Glyceraldehyde-3-phosphate dehydrogenase was 4.6% (0.1-43) prior to STI571 therapy and 5.3% (0.07-100) at the time of resistance (ns). 3/21 pts. showed a >10fold Bcr-Abl overexpression; (ii) genomic amplification of Bcr-Abl was found in 2/21 pts. investigated by IP-FISH; (iii) additional chromosomal aberrations resulting in clonal evolution were observed in 7/23 pts., of whom five developed aneuploidy; (iv) point mutations of the ATP binding site of the Abl tyrosine kinase domain were detected in 6/40 pts.. Mutations lead to amino acid substitutions (Y253F, n=1; E255K, n=2; E255V, n=1; T315I, n=2) which changed the conformation of the binding site. Mutations have been confirmed by DNA restriction digest analysis and excluded in pretherapeutic samples. Reactivation of Bcr-Abl was confirmed by Crkl immunoblotting in five pts. with point mutations demonstrating insensitivity to STI571 with a median proportion of phosphorylated Crkl of 63% (range 38-77%). The Abl autophosphorylation assay demonstrated an increase of the IC₅₀ for STI571 from 0.025 µM for wildtype Abl to 0.5 µM for Y253F, 0.4 µM for E255K, >0.5 µM for E255V, and 0.30 µM for T315I.

Further studies revealed the presence of additional mutations, i.e. Leu248Val, Glu252His, Tyr253His, Met351Thr, Glu355Gly, and His396Arg in STI571-resistant/refractory patients suffering from Ph chromosome-positive leukemia.

### Example 6:

### Materials and methods

### Patients

Seventy-one CML patients resistant or intolerant to Interferon alpha (INF-α) were treated with STI571 in 3 multicenter phase 2 trials. After 3 months of therapy, 34 of them showed hematological and cytogenetic response to STI571 (i.e. normal blood counts and greater than 65% of Ph-positive mitoses), whereas 29 showed no cytogenetic response. Twenty-four of them, including 16 in chronic phase and 8 in accelerated phase, were analyzed for Abl gene mutation. No material was available for the 5 remaining patients.

### RT-PCR-RFLP assay

RNA extraction. Total RNA was extracted from frozen aliquots of 107 Peripheral Blood Leucocytes with Trizol reagent (Life Technologies, UK) according to the manufacturer's instructions. RNA pellets were resuspended in 10 µl of RNAse-free water and quantity was estimated by ultraviolet spectrofluorometry.

*Reverse transcription.* cDNA was synthesized from 1 µg of total RNA in a 20 µl reaction mixture as previously described [Morschhauser F. et al., J. Clin. Oncology 18, 788-794 (2000)].

*PCR amplification* of a 412 bp fragment was performed with 2 µl of cDNA (corresponding to 100 ng of total RNA), 1X TaqGold reaction buffer (Applied Biosystem, USA), 1.5 mM MgCl₂, 250 µM each dATP, dCTP, dGTP, dTTP (Pharmacia, Sweden), 0.5U of AmpliTaq Gold polymerase (Applied Biosystem, USA) and 50 pmol of primer F2: 5'-GAG GGC GTG TGG AAG AAA TA-3' and R2: 5'-GCT GTG TAG GTG TCC CCT GT-3'. Thermocycling conditions used were 12 minutes at 94°C followed by 35 cycles of denaturation at 94°C for 1 minute, annealing at 57°C for 1 minute, extension at 72°C for 1 minute and a final extension step of 5 minutes at 72°C.

*RFLP analysis.* 1/5 of PCR product was digested by 5U of restriction enzyme *Dde* I (Roche, Switzerland) and electrophoresed on 2.5% Ethidium Bromide stained agarose gel.

### DNA extraction

Genomic DNA was extracted from 5.10⁶ Peripheral Blood Mononuclear Cells (PBMCs) using QIAmp DNA minikit (Qiagen, Germany) according to the manufacturer's recommendations. Quantity was estimated by ultraviolet spectrofluorometry.

### Sequence analysis

The whole kinase and ATP-loop Abl domain (amino acid 242 to 395) was amplified on cDNA in reaction mixture and PCR conditions as described above, using forward primer F3: 5'-CAT CAC CAT GAA GCA CAA GC-3' and reverse primer R2 at 60°C for annealing. After purification on QIAquick PCR purification column (Qiagen, Germany), 462 bp PCR fragments were sequenced following the ABI protocol for Taq-Dye Terminator Sequencing on an automated ABI377 sequencer. Sequences were analyzed with the Sequence Analysis software V3.3 and the Sequence Navigator software V1.0.1 (Applied Biosystem, USA). Sequencing was performed on both strands.

Detected mutations were always confirmed by sequencing both strands of 207 bp PCR products from DNA. PCR conditions are described above, using forward primer F4: 5'-GTC CTC GTT GTC TTG TTG GC-3' and reverse primer R4: 5'-CCC CTA CCT GTG GAT GAA GT-3' at 60°C for annealing.

### ASO-PCR assays

Mutated or wild-type sequences were specifically amplified in a non competitive PCR reaction performed on DNA in 50 µl reaction mixture and PCR conditions as described above, using allele-specific and reverse primers as follows: for the Thr315Ile mutation, F315C: 5'-GCC CCC GTT CTA TAT CAT CAC-3' or F315T: 5'-CCC GTT CTA TAT CAT CAT-3' and R1: 5'-GGA TGA AGT TTT TCT TCT CCA G-3' (annealing at 64°C; 158 bp PCR product); for the Phe311 Leu mutation, F311 T: 5'-CAC CCG GGA GCC CCC GT-3' or F311 C: 5'-CAC CCG GGA GCC CCC GC-3' and R4 (annealing at 64°C; 174 bp PCR fragment); for the Met351Thr mutation, F351T: 5'-CCA CTC AGA TCT CGT CAG CCA T-3' or F351 C: 5'-CCA CTC AGA TCT CGT CAG CCA C-3' and R5: 5'-GCC CTG AGA CCT CCT AGG CT-3' (annealing at 68 °C; 112 bp PCR fragment).

The sensitivity of this assay was determined for each mutation by amplification of 10-fold limited dilutions of 100 ng of patient's DNA at time of resistance in 100 ng of healthy control DNA.

### Results

### Analysis of Thr315Ile mutation

The Thr315Ile mutation was investigated by studying the loss of *Dde* I restriction enzyme site induced by C to T base change in the 24 STI571 resistant patients. Analysis was performed after cDNA amplification of a 412 bp PCR fragment at diagnosis and at the time of resistance.

In 3 CML patients in accelerated phase with resistance to STI571, the *Dde* I restricted pattern showed two populations of Abl transcripts, a wild-type sequence characterized by 2 fragments of 171 and 36 bp, respectively, and a mutated sequence characterized by a 207 bp uncut fragment. Differences in band intensities suggested a minor proportion of mutated transcript for one patient and a major proportion of mutated transcript for the two other patients. This RT-PCR-RFLP assay failed to detect Thr315Ile mutated transcript at diagnosis as only a 207 bp uncut fragment was detected in those patients.

Analysis was also performed on 16 patients with complete cytogenetic remission after 3 months of therapy with STI571. None of those patients presented the Thr315Ile mutation.

### Direct sequence analysis on DNA and RNA

In the 24 STI571 resistant patients, the Abl kinase domain and ATP-loop region were directly sequenced from PCR DNA and cDNA products (a 207 bp F4R4-PCR fragment and a 462 bp F3R2-PCR fragment, respectively) at the time of resistance and prior to STI571 therapy.

Sequencing data confirmed the Thr315Ile mutation in 2 of the 3 previously RT-PCR-RFLP detected patients, but failed in the remaining patient who presented a lower level of mutated transcript. The heterozygous rate for each patient is presented by comparison of specific C and T signal ranges on chromatographic primary sequence data, accordingly to RT-PCR-RFLP pattern.

Two of the 21 remaining patients showed 2 previously unreported mutations: one patient in accelerated phase after 12 months of STI571 therapy had a Phe311Leu substitution induced by a T to C base change, and one patient in chronic phase after 18 months of STI571 therapy had a Met351 Thr mutation induced by a T to C base change.

No mutation affecting the Glu255 amino acid was detected by this direct sequencing method.

### ASO-PCR monitoring

In order to increase the sensitivity of mutation detection, ASO-PCR assays were developed. The ASO-PCR monitoring showed that in patients having Thr315Ile, Phe311 Leu or Met351Thr mutations, these mutations were present prior to STI571 treatment, providing evidence that those point mutations preexisted to STI571 treatment. An increased proportion of mutated cells over time is shown by PCR signal intensities on Ethidium Bromide stained agarose gel in the 3 analyzed mutations. This last result strongly suggests clonal selection by functional STI571 resistance of mutated cells during therapy. As specific PCR products of mutated Abl gene were detected even after a 10000-fold dilution range, a very sensitive ASO-PCR test was developed: assuming that 100 ng of DNA represent approximately 15000 cells, it was possible to detect 1.5 Abl mutated cell in 15000 wild-type cells. As expected, for each point dilution, signal intensity of non-mutated cells remained constant. The strong specificity of the assay was demonstrated for each mutation by lack of mutated detected Abl sequence from healthy DNA controls.

### Example 7:

In this example we studied 43 CML patients (18 in chronic phase and 25 in advanced phase of the disease) who become either cytogenetical or hematological resistant to imatinib mesylate treatment. The advanced phase patients were treated with imatinib mesylate during a median time of 3 months and chronic phase patients during a median time of 13 months. After a first amplification including *BCR-ABL* fusion (1300pb), we carried out nested PCR to amplify a fragment of 584pb including the ATP binding domain and a fragment of 386pb containing the SH2 and SH3 domains. PCR products were sequenced allowing to cover the different ABL domains including amino acid 72 to 180 and amino acid 234 to 396 to study respectively SH2/SH3 and ATP binding domains.

Regarding the ATP binding domain among the 43 patients, 5 cases of mutations were detected. Three patients (one in accelerated phase, and 2 in blast crisis) exhibited the T3151 mutation. In one other accelerated phase patient the E255K mutation was detected. In a cytogenetic resistant patient (in chronic phase) treated by imatinib mesylatemore than one year, the investigations found a newly described Gly250Ala substitution. Regarding the SH2 and SH3 domains no mutation were detected in the different samples providing from the all the 43 CML patients studied.

Our data confirm that in CML patients treated with STI571, ABL mutations are not restricted to the accelerated phase of the disease and we report a new point mutation not described before.

After in vitro mutagenesis, Ba/F3 cell lines were engineered to express either wild-type and T3151, E255K, A250G mutants, in the aim to study the differential sensitivity to imatinib mesylate. Preliminary results confirmed the high level of resistance from the BaF/BCR-ABL*T315I. The other functional studies are in progress in the laboratory and be will compared to this one.

### Thus the present application provides

In the first aspect, an isolated polypeptide which comprises a functional kinase domain comprising the amino acid sequence of the native human Abl kinase domain or an essentially similar sequence thereof in which at least one amino acid selected from Met244, Leu248, Gly250, Glu252, Tyr253, Val256, Glu258, Phe311, Ile313, Phe317, Met318, Met351, Glu355, Glu359, Ile360, His361, Leu370, Asp381, Phe382, His396, Ser417, Glu459 and Phe486 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

In the second aspect, an isolated polypeptide according to the first aspect, wherein in the amino acid sequence of the native human Abl kinase domain or an essentially similar sequence thereof at least one amino acid selected from Met244, Leu248, Gly250, Glu252, Tyr253, Val256, Glu258, Phe311, Ile313, Phe317, Met318, Glu355, Glu359, Ile360, His361, Leu370, Asp381, Phe382, His396, Ser417, Glu459 and Phe486 is replaced by another amino acid.

In the third aspect, an isolated polypeptide according to the first aspect, wherein in the amino acid sequence of the native human Abl kinase domain or an essentially similar sequence thereof at least one amino acid selected from Met244, Leu248, Gly250, Glu252, Tyr253, Glu258, Phe311, Phe317, Met351, Glu355, Glu359, His396, Ser417, Glu459 and Phe486 is replaced by another amino acid.

In the fourth aspect, sn isolated polypeptide according to the third aspect, wherein in the amino acid sequence of the native human Abl kinase domain or an essentially similar sequence thereof at least one amino acid selected from Met244, Leu248, Gly250, Glu252, Tyr253, Glu258, Phe311, Phe317, Glu355, Glu359, His396, Ser417, Glu459 and Phe486 is replaced by another amino acid.

In the fifth aspect, an isolated polypeptide according to the fourth aspect, wherein in the amino acid sequence of the native human Abl kinase domain or an essentially similar sequence thereof at least one amino acid selected from Gly250, Tyr253, Glu258, Phe317 and His396 is replaced by another amino acid.

In the sixth aspect, an isolated polypeptide according to the any one of the above aspects, wherein in the amino acid sequence of the native human Abl kinase domain or an essentially similar sequence thereof a single amino acid is replaced by another amino acid.

In the seventh aspect, an isolated polypeptide according to the third aspect, wherein the amino acid sequence of the native human Abl kinase domain or an essentially similar sequence thereof contains at least one amino acid mutation selected from Met244Val, Leu248Val, Gly250Glu, Gly250Al, Glu252His, Tyr253His, Tyr253Phe, Glu258Gly, Phe311Leu, Phe317Leu, Met351Thr, Glu355Gly, Glu359Val, His396Pro, His396Arg, Ser417Tyr, Glu459Lys and Phe486Ser.

In the eighth aspect, an isolated polypeptide according to the seventh asepct, wherein the amino acid sequence of the native human Abl kinase domain or an essentially similar sequence thereof contains at least one amino acid mutation selected from Met244Val, Leu248Val, Gly250Glu, Gly250Al, Glu252His, Tyr253His, Tyr253Phe, Glu258Gly, Phe311Leu, Phe317Leu, Glu355Gly, Glu359Val, His396Pro, His396Arg, Ser417Tyr, Glu459Lys and Phe486Ser.

In the ninth aspect, an isolated polypeptide according to the eighth aspect, wherein the amino acid sequence of the native human Abl kinase domain or an essentially similar sequence thereof contains at least one amino acid mutation selected from Gly250Glu, Tyr253His, Tyr253Phe, Glu258Gly, Phe317Leu and His396Pro.

In the tenth aspect, an isolated polypeptide according to the seventh aspect, wherein the amino acid sequence of the native human Abl kinase domain or an essentially similar sequence thereof contains the amino acid mutation Met351Thr.

In the eleventh aspect, an isolated polypeptide according to the eighth aspect, wherein the amino acid sequence of the native human Abl kinase domain or an essentially similar sequence thereof contains the amino acid mutation Met244Val.

In the twelfth aspect, an isolated polypeptide according to the ninth aspect, wherein the amino acid sequence of the native human Abl kinase domain or an essentially similar sequence thereof contains the amino acid mutation Gly250Glu.

In the thirteenth aspect, an isolated polypeptide according to the ninth aspect, wherein the amino acid sequence of the native human Abl kinase domain or an essentially similar sequence thereof contains the amino acid mutation Tyr253His.

In the fourteenth aspect, an isolated polypeptide according to the ninth aspect, wherein the amino acid sequence of the native human Abl kinase domain or an essentially similar sequence thereof contains the amino acid mutation Tyr253Phe.

In the fifteenth aspect, an isolated polypeptide according to the ninth aspect, wherein the amino acid sequence of the native human Abl kinase domain or an essentially similar sequence thereof contains the amino acid mutation Glu258Gly.

In the sixteenth aspect, an isolated polypeptide according to the eighth aspect, wherein the amino acid sequence of the native human Abl kinase domain or an essentially similar sequence thereof contains the amino acid mutation Phe311 Leu.

In the seventeenth aspect, an isolated polypeptide according to the ninth aspect, wherein the amino acid sequence of the native human Abl kinase domain or an essentially similar sequence thereof contains the amino acid mutation Phe317Leu.

In the eighteenth aspect, an isolated polypeptide according to the ninth aspect, wherein the amino acid sequence of the native human Abl kinase domain or an essentially similar sequence thereof contains the amino acid mutation His396Pro.

In the nineteenth aspect, an isolated polypeptide according to the eighth aspect, wherein the amino acid sequence of the native human Abl kinase domain or an essentially similar sequence thereof contains the amino acid mutation Phe486Ser.

In the twentieth aspect, an isolated polypeptide according to the eighth aspect, wherein the amino acid sequence of the native human Abl kinase domain or an essentially similar sequence thereof contains the amino acid mutation Leu248Val.

In the twenty-first aspect, an isolated polypeptide according to the eighth aspect, wherein the amino acid sequence of the native human Abl kinase domain or an essentially similar sequence thereof contains the amino acid mutation Gly250Ala.

In the twenty-second aspect, a n isolated polypeptide according to the eighth aspect, wherein the amino acid sequence of the native human Abl kinase domain or an essentially similar sequence thereof contains the amino acid mutation Glu252His.

In the twenty-third aspect, an isolated polypeptide according to the eighth aspect, wherein the amino acid sequence of the native human Abl kinase domain or an essentially similar sequence thereof contains the amino acid mutation Glu355Gly.

In the twenty-fourth aspect, an isolated polypeptide according to the eighth aspect, wherein the amino acid sequence of the native human Abl kinase domain or an essentially similar sequence thereof contains the amino acid mutation Glu359Val.

In the twenty-fifth aspect, an isolated polypeptide according to the eighth aspect, wherein the amino acid sequence of the native human Abl kinase domain or an essentially similar sequence thereof contains the amino acid mutation His396Arg.

In the twenty-sixth aspect, an isolated polypeptide according to the eighth aspect, wherein the amino acid sequence of the native human Abl kinase domain or an essentially similar sequence thereof contains the amino acid mutation Ser417Tyr.

In the twenty-seventh aspect, an isolated polypeptide according to the eighth aspect, wherein the amino acid sequence of the native human Abl kinase domain or an essentially similar sequence thereof contains the amino acid mutation Glu459Lys.

In the twenty-eighth aspect, an isolated polypeptide according to any one of aspect from tenth to twenty-seventh, wherein the amino acid sequence of the native human Abl kinase domain may contain at least one additional amino acid mutation selected from Met244Val, Leu248Val, Gly250Glu, Gly250Al, Glu252His, Tyr253His, Tyr253Phe, Glu258Gly, Phe311Leu, Phe317Leu, Met351Thr, Glu355Gly, Glu359Val, His396Pro, His396Arg, Ser417Tyr, Glu459Lys and Phe486Ser.

In the twenty-ninth aspect, an isolated polypeptide according to any one of the above aspects, wherein the amino acid sequence of the native human Abl kinase domain consists of amino acids 229-500 of SEQ ID NO:2.

In the thirtieth aspect, an isolated polypeptide according to any one of the above aspects, which is a Bcr-Abl tyrosine kinase.

In the thirty-first aspect, an use of a polypeptide according to any one of the above aspects, to screen for compounds which inhibit the tyrosine kinase activity of said polypeptide.

In the thirty-second aspect, an isolated nucleic acid molecule comprising a nucleotide sequence that encodes a polypeptide according to any one of aspect first to thirtieth.

In the thirty-third aspect, an use of a nucleic acid molecule according to the thirty-second aspect in the production of a polypeptide according to any one of aspect first to the thirtieth for use in screening for compounds which inhibit the tyrosine kinase activity of said polypeptide.

In the thirty-forth aspect, a recombinant vector comprising a nucleic acid molecule according to the thirty second aspect.

In the thirty-fifth aspect, a recombinant vector according to the thirty-forth aspect, which is a recombinant expression vector.

In the thirty-sixth aspect. A host cell comprising a recombinant vector according to the thirty-forth or thirty-fifth aspect.

atg ttg gag atc tgc ctg aag ctg gtg ggc tgc aaa tcc aag aag ggg 48
Met Leu Glu Ile Cys Leu Lys Leu Val Gly Cys Lys Ser Lys Lys Gly
1               5                   10                  15

ctg tcc tcg tcc tcc agc tgt tat ctg gaa gaa gcc ctt cag cgg cca 96
Leu Ser Ser Ser Ser Ser Cys Tyr Leu Glu Glu Ala Leu Gln Arg Pro
            20                  25                  30

gta gca tct gac ttt gag cct cag ggt ctg agt gaa gcc gct cgt tgg 144
Val Ala Ser Asp Phe Glu Pro Gln Gly Leu Ser Glu Ala Ala Arg Trp
        35                  40                  45

aac tcc aag gaa aac ctt ctc gct gga ccc agt gaa aat gac ccc aac 192
Asn Ser Lys Glu Asn Leu Leu Ala Gly Pro Ser Glu Asn Asp Pro Asn
    50                  55                  60

ctt ttc gtt gca ctg tat gat ttt gtg gcc agt gga gat aac act cta 240
Leu Phe Val Ala Leu Tyr Asp Phe Val Ala Ser Gly Asp Asn Thr Leu
65                  70                  75                  80

agc ata act aaa ggt gaa aag ctc cgg gtc tta ggc tat aat cac aat 288
Ser Ile Thr Lys Gly Glu Lys Leu Arg Val Leu Gly Tyr Asn His Asn
                85                  90                  95

ggg gaa tgg tgt gaa gcc caa acc aaa aat ggc caa ggc tgg gtc cca 336
Gly Glu Trp Cys Glu Ala Gln Thr Lys Asn Gly Gln Gly Trp Val Pro
            100                 105                 110

agc aac tac atc acg cca gtc aac agt ctg gag aaa cac tcc tgg tac 384
Ser Asn Tyr Ile Thr Pro Val Asn Ser Leu Glu Lys His Ser Trp Tyr

115                 120                 125

cat ggg cct gtg tcc cgc aat gcc gct gag tat ctg ctg agc agc ggg        432
His Gly Pro Val Ser Arg Asn Ala Ala Glu Tyr Leu Leu Ser Ser Gly
    130                 135                 140

atc aat ggc agc ttc ttg gtg cgt gag agt gag agc agt cct ggc cag        480
Ile Asn Gly Ser Phe Leu Val Arg Glu Ser Glu Ser Ser Pro Gly Gln
145                 150                 155                 160

agg tcc atc tcg ctg aga tac gaa ggg agg gtg tac cat tac agg atc        528
Arg Ser Ile Ser Leu Arg Tyr Glu Gly Arg Val Tyr His Tyr Arg Ile
                165                 170                 175

aac act gct tct gat ggc aag ctc tac gtc tcc tcc gag agc cgc ttc        576
Asn Thr Ala Ser Asp Gly Lys Leu Tyr Val Ser Ser Glu Ser Arg Phe
            180                 185                 190

aac acc ctg gcc gag ttg gtt cat cat cat tca acg gtg gcc gac ggg        624
Asn Thr Leu Ala Glu Leu Val His His His Ser Thr Val Ala Asp Gly
        195                 200                 205

ctc atc acc acg ctc cat tat cca gcc cca aag cgc aac aag ccc act        672
Leu Ile Thr Thr Leu His Tyr Pro Ala Pro Lys Arg Asn Lys Pro Thr
    210                 215                 220

gtc tat ggt gtg tcc ccc aac tac gac aag tgg gag atg gaa cgc acg        720
Val Tyr Gly Val Ser Pro Asn Tyr Asp Lys Trp Glu Met Glu Arg Thr
225                 230                 235                 240

gac atc acc atg aag cac aag ctg ggc ggg ggc cag tac ggg gag gtg        768
Asp Ile Thr Met Lys His Lys Leu Gly Gly Gly Gln Tyr Gly Glu Val
                245                 250                 255

tac gag ggc gtg tgg aag aaa tac agc ctg acg gtg gcc gtg aag acc        816
Tyr Glu Gly Val Trp Lys Lys Tyr Ser Leu Thr Val Ala Val Lys Thr
            260                 265                 270
ttg aag gag gac acc atg gag gtg gaa gag ttc ttg aaa gaa gct gca        864
Leu Lys Glu Asp Thr Met Glu Val Glu Glu Phe Leu Lys Glu Ala Ala
        275                 280                 285

gtc atg aaa gag atc aaa cac cct aac ctg gtg cag ctc ctt ggg gtc        912
Val Met Lys Glu Ile Lys His Pro Asn Leu Val Gln Leu Leu Gly Val
    290                 295                 300

tgc acc cgg gag ccc ccg ttc tat atc atc act gag ttc atg acc tac        960
Cys Thr Arg Glu Pro Pro Phe Tyr Ile Ile Thr Glu Phe Met Thr Tyr
305                 310                 315                 320

ggg aac ctc ctg gac tac ctg agg gag tgc aac cgg cag gag gtg aac       1008
Gly Asn Leu Leu Asp Tyr Leu Arg Glu Cys Asn Arg Gln Glu Val Asn
                325                 330                 335

gcc gtg gtg ctg ctg tac atg gcc act cag atc tcg tca gcc atg gag       1056
Ala Val Val Leu Leu Tyr Met Ala Thr Gln Ile Ser Ser Ala Met Glu
            340                 345                 350

tac ctg gag aag aaa aac ttc atc cac aga gat ctt gct gcc cga aac       1104
Tyr Leu Glu Lys Lys Asn Phe Ile His Arg Asp Leu Ala Ala Arg Asn
        355                 360                 365

tgc ctg gta ggg gag aac cac ttg gtg aag gta gct gat ttt ggc ctg 1152
Cys Leu Val Gly Glu Asn His Leu Val Lys Val Ala Asp Phe Gly Leu
    370                 375                 380

agc agg ttg atg aca ggg gac acc tac aca gcc cat gct gga gcc aag 1200
Ser Arg Leu Met Thr Gly Asp Thr Tyr Thr Ala His Ala Gly Ala Lys
385                 390                 395                 400

ttc ccc atc aaa tgg act gca ccc gag agc ctg gcc tac aac aag ttc 1248
Phe Pro Ile Lys Trp Thr Ala Pro Glu Ser Leu Ala Tyr Asn Lys Phe
                405                 410                 415

tcc atc aag tcc gac gtc tgg gca ttt gga gta ttg ctt tgg gaa att 1296
Ser Ile Lys Ser Asp Val Trp Ala Phe Gly Val Leu Leu Trp Glu Ile
            420                 425                 430

gct acc tat ggc atg tcc cct tac ccg gga att gac ctg tcc cag gtg 1344
Ala Thr Tyr Gly Met Ser Pro Tyr Pro Gly Ile Asp Leu Ser Gln Val
        435                 440                 445
tat gag ctg cta gag aag gac tac cgc atg gag cgc cca gaa ggc tgc 1392
Tyr Glu Leu Leu Glu Lys Asp Tyr Arg Met Glu Arg Pro Glu Gly Cys
    450                 455                 460

cca gag aag gtc tat gaa ctc atg cga gca tgt tgg cag tgg aat ccc 1440
Pro Glu Lys Val Tyr Glu Leu Met Arg Ala Cys Trp Gln Trp Asn Pro
465                 470                 475                 480

tct gac cgg ccc tcc ttt gct gaa atc cac caa gcc ttt gaa aca atg 1488
Ser Asp Arg Pro Ser Phe Ala Glu Ile His Gln Ala Phe Glu Thr Met
                485                 490                 495

ttc cag gaa tcc agt atc tca gac gaa gtg gaa aag gag ctg ggg aaa 1536
Phe Gln Glu Ser Ser Ile Ser Asp Glu Val Glu Lys Glu Leu Gly Lys
            500                 505                 510

caa ggc gtc cgt ggg gct gtg agt acc ttg ctg cag gcc cca gag ctg 1584
Gln Gly Val Arg Gly Ala Val Ser Thr Leu Leu Gln Ala Pro Glu Leu
        515                 520                 525

ccc acc aag acg agg acc tcc agg aga gct gca gag cac aga gac acc 1632
Pro Thr Lys Thr Arg Thr Ser Arg Arg Ala Ala Glu His Arg Asp Thr
    530                 535                 540

act gac gtg cct gag atg cct cac tcc aag ggc cag gga gag agc gat 1680
Thr Asp Val Pro Glu Met Pro His Ser Lys Gly Gln Gly Glu Ser Asp
545                 550                 555                 560

cct ctg gac cat gag cct gcc gtg tct cca ttg ctc cct cga aaa gag 1728
Pro Leu Asp His Glu Pro Ala Val Ser Pro Leu Leu Pro Arg Lys Glu
                565                 570                 575

cga ggt ccc ccg gag ggc ggc ctg aat gaa gat gag cgc ctt ctc ccc 1776
Arg Gly Pro Pro Glu Gly Gly Leu Asn Glu Asp Glu Arg Leu Leu Pro
            580                 585                 590

aaa gac aaa aag acc aac ttg ttc agc gcc ttg atc aag aag aag aag 1824
Lys Asp Lys Lys Thr Asn Leu Phe Ser Ala Leu Ile Lys Lys Lys Lys
        595                 600                 605

aag aca gcc cca acc cct ccc aaa cgc agc agc tcc ttc cgg gag atg 1872
Lys Thr Ala Pro Thr Pro Pro Lys Arg Ser Ser Ser Phe Arg Glu Met
    610                 615                 620
gac ggc cag ccg gag cgc aga ggg gcc ggc gag gaa gag ggc cga gac 1920
Asp Gly Gln Pro Glu Arg Arg Gly Ala Gly Glu Glu Glu Gly Arg Asp
625                 630                 635                 640

atc agc aac ggg gca ctg gct ttc acc ccc ttg gac aca gct gac cca 1968
Ile Ser Asn Gly Ala Leu Ala Phe Thr Pro Leu Asp Thr Ala Asp Pro
                645                 650                 655

gcc aag tcc cca aag ccc agc aat ggg gct ggg gtc ccc aat gga gcc 2016
Ala Lys Ser Pro Lys Pro Ser Asn Gly Ala Gly Val Pro Asn Gly Ala
            660                 665                 670

ctc cgg gag tcc ggg ggc tca ggc ttc cgg tct ccc cac ctg tgg aag 2064
Leu Arg Glu Ser Gly Gly Ser Gly Phe Arg Ser Pro His Leu Trp Lys
        675                 680                 685

aag tcc agc acg ctg acc agc agc cgc cta gcc acc ggc gag gag gag 2112
Lys Ser Ser Thr Leu Thr Ser Ser Arg Leu Ala Thr Gly Glu Glu Glu
    690                 695                 700

ggc ggt ggc agc tcc agc aag cgc ttc ctg cgc tct tgc tcc gcc tcc 2160
Gly Gly Gly Ser Ser Ser Lys Arg Phe Leu Arg Ser Cys Ser Ala Ser
705                 710                 715                 720

tgc gtt ccc cat ggg gcc aag gac acg gag tgg agg tca gtc acg ctg 2208
Cys Val Pro His Gly Ala Lys Asp Thr Glu Trp Arg Ser Val Thr Leu
                725                 730                 735

cct cgg gac ttg cag tcc acg gga aga cag ttt gac tcg tcc aca ttt 2256
Pro Arg Asp Leu Gln Ser Thr Gly Arg Gln Phe Asp Ser Ser Thr Phe
            740                 745                 750

gga ggg cac aaa agt gag aag ccg gct ctg cct cgg aag agg gca ggg 2304
Gly Gly His Lys Ser Glu Lys Pro Ala Leu Pro Arg Lys Arg Ala Gly
        755                 760                 765

gag aac agg tct gac cag gtg acc cga ggc aca gta acg cct ccc ccc 2352
Glu Asn Arg Ser Asp Gln Val Thr Arg Gly Thr Val Thr Pro Pro Pro
    770                 775                 780

agg ctg gtg aaa aag aat gag gaa gct gct gat gag gtc ttc aaa gac 2400
Arg Leu Val Lys Lys Asn Glu Glu Ala Ala Asp Glu Val Phe Lys Asp
785                 790                 795                 800
atc atg gag tcc agc ccg ggc tcc agc ccg ccc aac ctg act cca aaa 2448
Ile Met Glu Ser Ser Pro Gly Ser Ser Pro Pro Asn Leu Thr Pro Lys
                805                 810                 815

ccc ctc cgg cgg cag gtc acc gtg gcc cct gcc tcg ggc ctc ccc cac 2496
Pro Leu Arg Arg Gln Val Thr Val Ala Pro Ala Ser Gly Leu Pro His
            820                 825                 830

aag gaa gaa gct gaa aag ggc agt gcc tta ggg acc cct gct gca gct 2544
Lys Glu Glu Ala Glu Lys Gly Ser Ala Leu Gly Thr Pro Ala Ala Ala
        835                 840                 845

gag cca gtg acc ccc acc agc aaa gca ggc tca ggt gca cca ggg ggc 2592
Glu Pro Val Thr Pro Thr Ser Lys Ala Gly Ser Gly Ala Pro Gly Gly

850                 855                 860

acc agc aag ggc ccc gcc gag gag tcc aga gtg agg agg cac aag cac      2640
Thr Ser Lys Gly Pro Ala Glu Glu Ser Arg Val Arg Arg His Lys His
865                 870                 875                 880

tcc tct gag tcg cca ggg agg gac aag ggg aaa ttg tcc agg ctc aaa      2688
Ser Ser Glu Ser Pro Gly Arg Asp Lys Gly Lys Leu Ser Arg Leu Lys
                885                 890                 895

cct gcc ccg ccg ccc cca cca gca gcc tct gca ggg aag gct gga gga      2736
Pro Ala Pro Pro Pro Pro Pro Ala Ala Ser Ala Gly Lys Ala Gly Gly
            900                 905                 910

aag ccc tcg cag agc ccg agc cag gag gcg gcc ggg gag gca gtc ctg      2784
Lys Pro Ser Gln Ser Pro Ser Gln Glu Ala Ala Gly Glu Ala Val Leu
        915                 920                 925

ggc gca aag aca aaa gcc acg agt ctg gtt gat gct gtg aac agt gac      2832
Gly Ala Lys Thr Lys Ala Thr Ser Leu Val Asp Ala Val Asn Ser Asp
    930                 935                 940

gct gcc aag ccc agc cag ccg gga gag ggc ctc aaa aag ccc gtg ctc      2880
Ala Ala Lys Pro Ser Gln Pro Gly Glu Gly Leu Lys Lys Pro Val Leu
945                 950                 955                 960

ccg gcc act cca aag cca cag tcc gcc aag ccg tcg ggg acc ccc atc      2928
Pro Ala Thr Pro Lys Pro Gln Ser Ala Lys Pro Ser Gly Thr Pro Ile
                965                 970                 975
agc cca gcc ccc gtt ccc tcc acg ttg cca tca gca tcc tcg gcc ctg      2976
Ser Pro Ala Pro Val Pro Ser Thr Leu Pro Ser Ala Ser Ser Ala Leu
            980                 985                 990

gca ggg gac cag ccg tct tcc act  gcc ttc atc cct ctc  ata tca acc      3024
Ala Gly Asp Gln Pro Ser Ser Thr  Ala Phe Ile Pro Leu  Ile Ser Thr
        995                 1000                1005

cga gtg  tct ctt cgg aaa acc  cgc cag cct cca gag  cgg atc gcc      3069
Arg Val  Ser Leu Arg Lys Thr  Arg Gln Pro Pro Glu  Arg Ile Ala
    1010                1015                1020

agc ggc  gcc atc acc aag ggc  gtg gtc ctg gac agc  acc gag gcg      3114
Ser Gly  Ala Ile Thr Lys Gly  Val Val Leu Asp Ser  Thr Glu Ala
    1025                1030                1035

ctg tgc  ctc gcc atc tct agg  aac tcc gag cag atg  gcc agc cac      3159
Leu Cys  Leu Ala Ile Ser Arg  Asn Ser Glu Gln Met  Ala Ser His
    1040                1045                1050

agc gca  gtg ctg gag gcc ggc  aaa aac ctc tac acg  ttc tgc gtg      3204
Ser Ala  Val Leu Glu Ala Gly  Lys Asn Leu Tyr Thr  Phe Cys Val
    1055                1060                1065

agc tat  gtg gat tcc atc cag  caa atg agg aac aag  ttt gcc ttc      3249
Ser Tyr  Val Asp Ser Ile Gln  Gln Met Arg Asn Lys  Phe Ala Phe
    1070                1075                1080

cga gag  gcc atc aac aaa ctg  gag aat aat ctc cgg  gag ctt cag      3294
Arg Glu  Ala Ile Asn Lys Leu  Glu Asn Asn Leu Arg  Glu Leu Gln
    1085                1090                1095

atc tgc ccg gcg aca gca ggc agt ggt ccg gcg gcc act cag gac 3339
Ile Cys Pro Ala Thr Ala Gly Ser Gly Pro Ala Ala Thr Gln Asp
    1100                1105                1110

ttc agc aag ctc ctc agt tcg gtg aag gaa atc agt gac ata gtg 3384
Phe Ser Lys Leu Leu Ser Ser Val Lys Glu Ile Ser Asp Ile Val
    1115                1120                1125

cag agg tag 3393
Gln Arg
    1130

Met Leu Glu Ile Cys Leu Lys Leu Val Gly Cys Lys Ser Lys Lys Gly
1 5 10 15

Leu Ser Ser Ser Ser Ser Cys Tyr Leu Glu Glu Ala Leu Gln Arg Pro
20 25 30

Val Ala Ser Asp Phe Glu Pro Gln Gly Leu Ser Glu Ala Ala Arg Trp
35 40 45

Asn Ser Lys Glu Asn Leu Leu Ala Gly Pro Ser Glu Asn Asp Pro Asn
50 55 60

Leu Phe Val Ala Leu Tyr Asp Phe Val Ala Ser Gly Asp Asn Thr Leu
65 70 75 80

Ser Ile Thr Lys Gly Glu Lys Leu Arg Val Leu Gly Tyr Asn His Asn
85 90 95

Gly Glu Trp Cys Glu Ala Gln Thr Lys Asn Gly Gln Gly Trp Val Pro
100 105 110

Ser Asn Tyr Ile Thr Pro Val Asn Ser Leu Glu Lys His Ser Trp Tyr
115 120 125

His Gly Pro Val Ser Arg Asn Ala Ala Glu Tyr Leu Leu Ser Ser Gly
130 135 140

Ile Asn Gly Ser Phe Leu Val Arg Glu Ser Glu Ser Ser Pro Gly Gln
145 150 155 160

Arg Ser Ile Ser Leu Arg Tyr Glu Gly Arg Val Tyr His Tyr Arg Ile
165 170 175

Asn Thr Ala Ser Asp Gly Lys Leu Tyr Val Ser Ser Glu Ser Arg Phe
180 185 190

Asn Thr Leu Ala Glu Leu Val His His His Ser Thr Val Ala Asp Gly
195 200 205

Leu Ile Thr Thr Leu His Tyr Pro Ala Pro Lys Arg Asn Lys Pro Thr
210 215 220

Val Tyr Gly Val Ser Pro Asn Tyr Asp Lys Trp Glu Met Glu Arg Thr
225 230 235 240

Asp Ile Thr Met Lys His Lys Leu Gly Gly Gly Gln Tyr Gly Glu Val
                245                 250                 255

Tyr Glu Gly Val Trp Lys Lys Tyr Ser Leu Thr Val Ala Val Lys Thr
            260                 265                 270

Leu Lys Glu Asp Thr Met Glu Val Glu Glu Phe Leu Lys Glu Ala Ala
        275                 280                 285

Val Met Lys Glu Ile Lys His Pro Asn Leu Val Gln Leu Leu Gly Val
    290                 295                 300

Cys Thr Arg Glu Pro Pro Phe Tyr Ile Ile Thr Glu Phe Met Thr Tyr
305                 310                 315                 320

Gly Asn Leu Leu Asp Tyr Leu Arg Glu Cys Asn Arg Gln Glu Val Asn
                325                 330                 335

Ala Val Val Leu Leu Tyr Met Ala Thr Gln Ile Ser Ser Ala Met Glu
            340                 345                 350

Tyr Leu Glu Lys Lys Asn Phe Ile His Arg Asp Leu Ala Ala Arg Asn
        355                 360                 365

Cys Leu Val Gly Glu Asn His Leu Val Lys Val Ala Asp Phe Gly Leu
    370                 375                 380

Ser Arg Leu Met Thr Gly Asp Thr Tyr Thr Ala His Ala Gly Ala Lys
385                 390                 395                 400

Phe Pro Ile Lys Trp Thr Ala Pro Glu Ser Leu Ala Tyr Asn Lys Phe
                405                 410                 415

Ser Ile Lys Ser Asp Val Trp Ala Phe Gly Val Leu Leu Trp Glu Ile
            420                 425                 430

Ala Thr Tyr Gly Met Ser Pro Tyr Pro Gly Ile Asp Leu Ser Gln Val
        435                 440                 445

Tyr Glu Leu Leu Glu Lys Asp Tyr Arg Met Glu Arg Pro Glu Gly Cys
    450                 455                 460

Pro Glu Lys Val Tyr Glu Leu Met Arg Ala Cys Trp Gln Trp Asn Pro
465                 470                 475                 480

Ser Asp Arg Pro Ser Phe Ala Glu Ile His Gln Ala Phe Glu Thr Met
                485                 490                 495

Phe Gln Glu Ser Ser Ile Ser Asp Glu Val Glu Lys Glu Leu Gly Lys
            500                 505                 510

Gln Gly Val Arg Gly Ala Val Ser Thr Leu Leu Gln Ala Pro Glu Leu
        515                 520                 525

Pro Thr Lys Thr Arg Thr Ser Arg Arg Ala Ala Glu His Arg Asp Thr
    530                 535                 540

Thr Asp Val Pro Glu Met Pro His Ser Lys Gly Gln Gly Glu Ser Asp

545                 550                 555                 560

Pro Leu Asp His Glu Pro Ala Val Ser Pro Leu Leu Pro Arg Lys Glu
                565                 570                 575

Arg Gly Pro Pro Glu Gly Gly Leu Asn Glu Asp Glu Arg Leu Leu Pro
            580                 585                 590

Lys Asp Lys Lys Thr Asn Leu Phe Ser Ala Leu Ile Lys Lys Lys Lys
        595                 600                 605

Lys Thr Ala Pro Thr Pro Pro Lys Arg Ser Ser Ser Phe Arg Glu Met
    610                 615                 620

Asp Gly Gln Pro Glu Arg Arg Gly Ala Gly Glu Glu Gly Arg Asp
625                 630                 635                 640

Ile Ser Asn Gly Ala Leu Ala Phe Thr Pro Leu Asp Thr Ala Asp Pro
                645                 650                 655

Ala Lys Ser Pro Lys Pro Ser Asn Gly Ala Gly Val Pro Asn Gly Ala
            660                 665                 670

Leu Arg Glu Ser Gly Gly Ser Gly Phe Arg Ser Pro His Leu Trp Lys
        675                 680                 685

Lys Ser Ser Thr Leu Thr Ser Ser Arg Leu Ala Thr Gly Glu Glu Glu
    690                 695                 700

Gly Gly Gly Ser Ser Ser Lys Arg Phe Leu Arg Ser Cys Ser Ala Ser
705                 710                 715                 720

Cys Val Pro His Gly Ala Lys Asp Thr Glu Trp Arg Ser Val Thr Leu
                725                 730                 735

Pro Arg Asp Leu Gln Ser Thr Gly Arg Gln Phe Asp Ser Ser Thr Phe
            740                 745                 750

Gly Gly His Lys Ser Glu Lys Pro Ala Leu Pro Arg Lys Arg Ala Gly
        755                 760                 765

Glu Asn Arg Ser Asp Gln Val Thr Arg Gly Thr Val Thr Pro Pro Pro
    770                 775                 780

Arg Leu Val Lys Lys Asn Glu Glu Ala Ala Asp Glu Val Phe Lys Asp
785                 790                 795                 800

Ile Met Glu Ser Ser Pro Gly Ser Ser Pro Pro Asn Leu Thr Pro Lys
                805                 810                 815

Pro Leu Arg Arg Gln Val Thr Val Ala Pro Ala Ser Gly Leu Pro His
            820                 825                 830

Lys Glu Glu Ala Glu Lys Gly Ser Ala Leu Gly Thr Pro Ala Ala Ala
        835                 840                 845

Glu Pro Val Thr Pro Thr Ser Lys Ala Gly Ser Gly Ala Pro Gly Gly
    850                 855                 860

Thr Ser Lys Gly Pro Ala Glu Glu Ser Arg Val Arg Arg His Lys His
865                 870                 875                 880

Ser Ser Glu Ser Pro Gly Arg Asp Lys Gly Lys Leu Ser Arg Leu Lys
                885                 890                 895

Pro Ala Pro Pro Pro Pro Pro Ala Ala Ser Ala Gly Lys Ala Gly Gly
            900                 905                 910

Lys Pro Ser Gln Ser Pro Ser Gln Glu Ala Ala Gly Glu Ala Val Leu
        915                 920                 925

Gly Ala Lys Thr Lys Ala Thr Ser Leu Val Asp Ala Val Asn Ser Asp
    930                 935                 940

Ala Ala Lys Pro Ser Gln Pro Gly Glu Gly Leu Lys Lys Pro Val Leu
945                 950                 955                 960

Pro Ala Thr Pro Lys Pro Gln Ser Ala Lys Pro Ser Gly Thr Pro Ile
                965                 970                 975

Ser Pro Ala Pro Val Pro Ser Thr Leu Pro Ser Ala Ser Ser Ala Leu
            980                 985                 990

Ala Gly Asp Gln Pro Ser Ser Thr  Ala Phe Ile Pro Leu  Ile Ser Thr
        995                 1000                 1005

Arg Val  Ser Leu Arg Lys Thr  Arg Gln Pro Pro Glu  Arg Ile Ala
    1010                 1015                 1020

Ser Gly  Ala Ile Thr Lys Gly  Val Val Leu Asp Ser  Thr Glu Ala
    1025                 1030                 1035

Leu Cys  Leu Ala Ile Ser Arg  Asn Ser Glu Gln Met  Ala Ser His
    1040                 1045                 1050

Ser Ala  Val Leu Glu Ala Gly  Lys Asn Leu Tyr Thr  Phe Cys Val
    1055                 1060                 1065

Ser Tyr  Val Asp Ser Ile Gln  Gln Met Arg Asn Lys  Phe Ala Phe
    1070                 1075                 1080

Arg Glu  Ala Ile Asn Lys Leu  Glu Asn Asn Leu Arg  Glu Leu Gln
    1085                 1090                 1095

Ile Cys  Pro Ala Thr Ala Gly  Ser Gly Pro Ala Ala  Thr Gln Asp
    1100                 1105                 1110

Phe Ser  Lys Leu Leu Ser Ser  Val Lys Glu Ile Ser  Asp Ile Val
    1115                 1120                 1125

Gln Arg
    1130

## Claims

1. An isolated polypeptide which comprises a functional kinase domain comprising the amino acid sequence of the native human Abl kinase domain or an essentially similar sequence thereof in which Met351 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

2. The isolated polypeptide according to claim 1, wherein Met351 is replaced by Threonine.

3. The isolated polypeptide according to claim 1 or 2, wherein the amino acid sequence of the native human Abl kinase domain consists of amino acids 229-500 of SEQ ID NO:2.

4. The isolated polypeptide according to any one of claims 1 to 3, which is a Bcr-Abl tyrosine kinase.

5. Use of a polypeptide according to any one of claims 1 to 4 to screen for compounds which inhibit the tyrosine kinase activity of said polypeptide.

6. An isolated nucleic acid molecule comprising a nucleotide sequence that encodes a polypeptide according to any one of claims 1 to 4.

7. Use of a nucleic acid molecule according to claim 6 in the production of a polypeptide according to any one of claims 1 to 4 for use in screening for compounds which inhibit the tyrosine kinase activity of said polypeptide.

8. A recombinant vector comprising a nucleic acid molecule according to claim 6.

9. The recombinant vector according to claim 8, which is a recombinant expression vector.

10. A host cell comprising a recombinant vector according to claim 8 or 9.

11. A method of identifying M351T mutation in a patient refractory or resistant to the treatment of N-[4-methyl-3-(4-pyridin-3-yl-pyrimidin-2-ylamino)-phenyl]-4-(4-methyl-piperazin-1-ylmethyl)-benzamide or a salt thereof, the method comprises amplifying and sequencing the nucleotide sequence of the Abl kinase domain of Bcr-Abl in said patient, and determining whether methionine at position 351 is replaced by Threonine.

12. The isolated polypeptide according to any one of claims 1 to 3, which contains a second BCR-ABL kinase domain mutation selected from the mutations provided in Table 7.

13. The isolated polypeptide according to claim 12, which is a Bcr-Abl tyrosine kinase.

14. Use of a polypeptide according to any one of claims 12 to 13 to screen for compounds which inhibit the tyrosine kinase activity of said polypeptide.

15. An isolated nucleic acid molecule comprising a nucleotide sequence that encodes a polypeptide according to any one of claims 12 to 13.

16. Use of a nucleic acid molecule according to claim 15 in the production of a polypeptide according to any one of claims 12 to 13 for use in screening for compounds which inhibit the tyrosine kinase activity of said polypeptide.
